# EUROPEAN PATENT SPECIFICATION

(11) **EP 2 132 185 B1**
(45) Date of publication and mention of the grant of the patent: **03.08.2011**
(21) Application number: 08737283.5
(22) Date of filing: 10.04.2008
(51) Int. Cl.: C07D 239/48, A61K 31/506, A61P 25/00

(54) **PYRIMIDINYL-PIPERAZINES USEFUL AS DOPAMINE D3/D2 RECEPTOR LIGANDS**
PYRIMIDINYL-PIPERAZINE ALS DOPAMIN D3/D2-REZEPTORLIGANDEN
PYRIMIDINYL-PIPÉRAZINES UTILES EN TANT QUE LIGANDS DES RÉCEPTEURS D3/D2 DE LA DOPAMINE

(30) Priority: 11.04.2007 HU 0700269
(43) Date of publication of application: 16.12.2009
(73) Proprietor: Richter Gedeon Nyrt., 1103 Budapest (HU)
(72) Inventor: BARTÁNÉ SZALAI, Gizella, H-1162 Budapest (HU); ÁGAINÉ CSONGOR, Éva, H-1031 Budapest (HU); DOMÁNY, György, H-7695 Óbánya (HU); GYERTYÁN, István, H-1161 Budapest (HU); KISS, Béla, H-1182 Budapest (HU); LASZY, Judit, H-2094 Nagykovácsi (HU); SÁGHY, Katalin, H-1082 Budapest (HU); SCHMIDT, Éva, H-2089 Telki (HU); FARKAS, Sándor, H-1103 Budapest (HU); KOMLÓDI, Zsolt, H-1203 Budapest (HU)
(74) Representative: HOFFMANN EITLE
(86) International application number: PCT/HU2008/000032
(87) International publication number: WO 2008/125891

(56) References cited:
- EP-A- 0 431 580
- WO-A-2005/012266
- WO-A-2007/148208
- US-A- 4 051 244
- CLAUDE GUÉRÉMY ET AL.: "2-amino-6-chloro-4-(N-methylpiperazino)- pyrimidines, inhibitors of spiroperidol binding" JOURNAL OF MEDICINAL CHEMISTRY., vol. 25, no. 12, 1982, pages 1459-1465, XP002502671 USAMERICAN CHEMICAL SOCIETY. WASHINGTON. cited in the application
- WUSTROW D ET AL: "Aminopyrimidines with High Affinity for Both Serotonin and Dopamine Receptors" JOURNAL OF MEDICINAL CHEMISTRY, US AMERICAN CHEMICAL SOCIETY. WASHINGTON, vol. 41, no. 5, 6 February 1998 (1998-02-06), pages 760-771, XP002378781 ISSN: 0022-2623

## Description

### FIELD OF THE INVENTION

The present invention relates to new dopamine D₃ and D₂ receptor subtype preferring ligands of formula (I) and/or geometric isomers and/or stereoisomers and/or diastereomers and/or salts and/or hydrates and/or solvates thereof. The invention also relates to processes for preparing the same, to compositions containing the same and to their use in the treatment and/or prevention of conditions which requires modulation of dopamine receptors.

### BACKGROUND OF THE INVENTION

Cyclohexane derivatives that are useful as therapeutics for the treatment of pain are described in International Patent Publication No. WO 99/67206.

Compounds containing a cyclohexane, pyrimidine and piperazine ring are described in European Patent No. EP 431,580 and U.S. Patent No. 4,957,921. These compounds act as central nervous system agents and dopaminergic agents, respectively. These compounds, however, do not contain an alkyl-amino group in the 2-position of the pyrimidine ring. Dopamine D₃ receptor modulator compounds containing a pyrimidine and piperazine ring are described in U.S. Patent Application Publication No. 2004/259882. These compounds do not, however, contain a cyclohexane ring.

WO 2005/012266 A and J. Med. Chem., Vol. 41, No. 5,1998, pages 760-771 also describe D2/D3 dopamine receptor ligands having a cyclohexyl-piperazinyl skeleton.

US-A-4 051 244 describes antipsychotic compounds having a 2-amino-5,6-di-halogenopyrimidin-4-yl group.
2-Amino-6-chloro-4-(N-methylpiperazino)pyrimidines as inhibitors of spiroperidol binding are described in, e.g., J. Med. Chem., 25, 1459, (1982).

### SUMMARY OF THE INVENTION

Surprisingly, it has been found that in contrast to the compounds described above, the compounds of formula (I) of the present invention have high or very high affinity for dopamine D₃ receptors, and moderate to high affinity for dopamine D₂ receptors always in such a combination that the D₃ affinity is 5 to 50 fold higher than the D₂ affinity. In addition, the compounds of the present invention show even higher selectivity over other receptors. For example, these compounds do not show affinity for alpha-1 adrenoceptors, i.e., their inhibitor constants (Ki) are higher or much higher than 1000 nM.

The dual (*i.e*. D₃ and D₂) receptor functional antagonism coupled in the above mentioned particular proportion is especially important as it allows the simultaneous manifestation of the beneficial modulation of both D₃ and D₂ receptors, however, without the appearance of the known disadvantages of each individual receptor action.

The compounds of formula (I) will be referred to in this application as "D₃/D₂ ligands".

The present invention relates to new piperazine derivatives of formula (I): 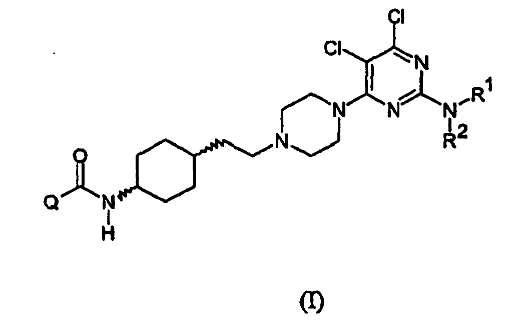 wherein
Q represents C₁₋₄ alkyl, -NR³R⁴, Phenyl, optionally substituted phenyl, 1-pyrrolidinyl, 1-piperidinyl, 4-R⁵-piperazin-1-yl or 4-morpholinyl group;
R¹ represents hydrogen or C₁₋₄ alkyl group;
R² represents hydrogen or C₁₋₄ alkyl group;
R³ represents hydrogen, C₁₋₄ alkyl group, phenyl or optionally substituted phenyl;
R⁴ represents hydrogen, C₁₋₄ alkyl group, phenyl or optionally substituted phenyl;
R⁵ represents hydrogen or C₁₋₄ alkyl group;
and/or geometric isomers and/or stereoisomers and/or diastereomers and/or salts and/or hydrates and/or solvates thereof, to processes for preparing the same, to pharmacological compositions containing the same and to their use in the treatment and/or prevention of pathological conditions which require the modulation of dopamine receptors, such as, but not limited to, psychoses (*e.g*. schizophrenia, schizo-affective disorders), drug (*e.g*. alcohol, cocaine, nicotine, opioids) abuse, cognitive impairment accompanying schizophrenia, mild-to-moderate cognitive deficits, dementia, psychotic states associated with dementia, eating disorders (*e.g*, bulimia nervosa, etc.), attention deficit disorders, hyperactivity disorders, psychotic depression, mania, bipolar disorder, paranoid and delusional disorders, dyskinetic disorders (*e.g*. Parkinson's disease, neuroleptic induced parkinsonism, tardive dyskinesia), depression and depressive states, anxiety disorders, sexual dysfunctions (eg. erectile dysfunctions), sleep disorders, emesis, aggression, autism and pain.

The present invention also relates to compounds of formula (III): 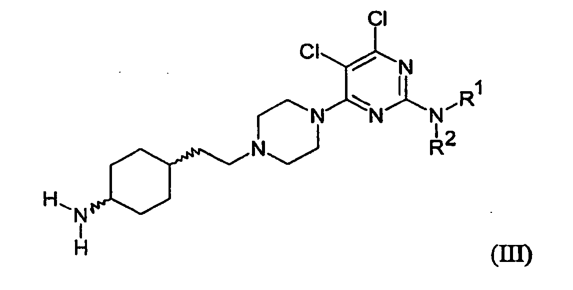 wherein
R¹ represents hydrogen or C₁₋₄ alkyl group, and
R² represents hydrogen or C₁₋₄ alkyl group.

### DETAILED DESCRIPTION OF THE INVENTION

In one aspect, the present invention relates to compounds of formula (I): 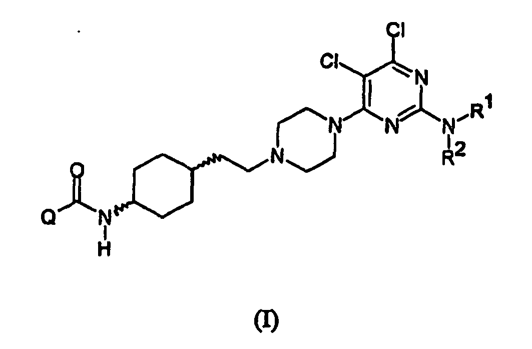 wherein
Q represents C₁₋₄ alkyl, -NR³R⁴, phenyl, optionally substituted phenyl, 1-pyrrolidinyl, 1-piperidinyl, 4-R⁵-piperazin-1-yl or 4-morpholinyl group;
R¹ represents hydrogen or C₁₋₄ alkyl group;
R² represents hydrogen or C₁₋₄ alkyl group;
R³ represents hydrogen, C₁₋₄ alkyl group, phenyl or optionally substituted phenyl;
R⁴ represents hydrogen, C₁₋₄ alkyl group, phenyl or optionally substituted phenyl;
R⁵ represents hydrogen or C₁₋₄ alkyl group;
and/or geometric isomers and/or stereoisomers and/or diastereomers and/or pharmaceutically acceptable salts and/or hydrates and/or solvates thereof.

The term "optionally substituted phenyl" as used herein means a phenyl group which can be substituted in any position by one or more halogen, C₁₋₄ alkyl, C₁₋₄ alkoxy, trifluoromethyl and/or cyano group, or combinations thereof

The present invention also relates to salts of compounds of formula (I) formed with acids.

Both organic and inorganic acids can be used for the formation of acid addition salts. Suitable inorganic acids include, but are not limited to, hydrochloric acid, sulfuric acid, nitric acid and phosphoric acid. Representatives of monovalent organic acids include, but are not limited to, formic acid, acetic acid, propionic acid, and different butyric acids, valeric acids and capric acids. Representatives of bivalent organic acids include, but are not limited to, oxalic acid, malonic acid, maleic acid, fumaric acid and succinic acid. Other organic acids can also be used, such as hydroxy acids, for example, citric acid, tartaric acid, or aromatic carboxylic acids, for example, benzoic acid or salicylic acid, as well as aliphatic and aromatic sulfonic acids, for example, methanesulfonic acid, naphtalenesulfonic acid and p-toluenesulfonic acid. A preferred group of acid addition salts are those in which the acid component itself is physiologically acceptable and does not have a therapeutic effect in the applied dose and/or it does not have unfavourable influence on the effect of the active ingredient. These acid addition salts are pharmaceutically acceptable acid addition salts. Acid addition salts which are not pharmaceutically acceptable acid addition salts can be advantageous in the purification and isolation of the desired compounds of formula (I), and are therefore also included within the scope of the present invention.

Solvates and/or hydrates of compounds of formula (I), as well as solvates and/or hydrates of salts of compounds of formula (1) are also included within the scope of the present invention.

One of ordinary skill in the art will recognize that compounds of Formula I can exist in different tautomeric and geometrical isomeric forms. For example, the compounds of formula (I) exist in the form of *cis* and *trans* isomers with respect to the configuration of the cyclohexane ring. The compounds of present invention are preferably in the *trans* configuration. In addition, certain compounds of formula (I) can exist as stereoisomers and diastereomers. All of these compounds, including *cis* isomers, *trans* isomers, diastereomic mixtures, racemates, nonracemic mixtures of enantiomers, substantially pure, and pure enantiomers, are within the scope of the present invention. Substantially pure enantiomers contain no more than 5% w/w of the corresponding opposite enantiomer, preferably no more than 2%, most preferably no more than 1%.

The optical isomers can be obtained by resolution of the racemic mixtures according to conventional processes, for example, by the formation of diastereoisomeric salts using an optically active acid or base or formation of covalent diastereomers. Examples of appropriate acids are tartaric, diacetyltartaric, dibenzoyltartaric, ditoluoyltartaric and camphorsulfonic acid. Mixtures of diastereoisomers can be separated into their individual diastereomers on the basis of their physical and/or chemical differences by methods known to those skilled in the art, for example, by chromatography or fractional crystallization. The optically active bases or acids are then liberated from the separated diastereomeric salts. A different process for separation of optical isomers involves the use of chiral chromatography (e.g., chiral HPLC columns), with or without conventional derivation, optimally chosen to maximize the separation of the enantiomers. Suitable chiral HPLC columns are manufactured by Diacel, e.g., Chiracel OD and Chiracel OJ among many others, all routinely selectable. Enzymatic separations, with or without derivitization, are also useful. The optically active compounds of formulas (I) can likewise be obtained by utilizing optically active starting materials in chiral synthesis processes under reaction conditions which do not cause racemization.

As used herein in the present specification and claims a "compound of formula (I)" will be deemed to encompass both the free base and salts, e.g., pharmaceutically acceptable salts, thereof.

In certain embodiments, preferred compounds of the invention are those compounds of formula (I) wherein
Q represents C₁₋₄ alkyl, NR³R⁴ or 4-morpholinyl group,
R¹ represents hydrogen or C₁₋₄ alkyl group;
R² represents hydrogen or C₁₋₄ alkyl group;
R³ represents hydrogen or C₁₋₄ alkyl group;
R⁴ represents hydrogen or C₁₋₄ alkyl group;
and/or geometric isomers and/or stereoisomers and/or diastereomers and/or salts and/or hydrates and/or solvates thereof.

In a further embodiment, the compound of formula (I) is selected from:
*trans*-N-(4-{2-[4-(5,6-dichloro-2-methylamino-pyrimidin-4-yl)piperazin-1-yl]-ethyl]-cyclohexyl}-acetamide,
*trans*-(4-{2-[4-(5,6-dichloro-2-methylamino-pyrimidin-4-yl)-piperazin-l-yl]-ethyl}-cyclohexyl)-urea,
*trans*-morpholine-4-carboxylic acid (4-{2-[4-(5,6-dichloro-2-ethylamino-pyrimidin-4-yl)-piperazin-1-yl]-ethyl}-cyclohexyl)-amide,
*trans*-(4-{2-[4-(5,6-dichloro-2-ethylamino-pyrimidin-4-yl)-piperazin-1-yl]-ethyl}-cyclohexyl)-urea,
*trans*-*N*-(4-(2-[4-(5,6-dichloro-2-dimethylamino-pyrimidin-4-yl)-piperazin-1-yl]-ethyl}-cyclohexyl)-acetamide,
*trans*-*N*-(4-{2-[4-(5,6-dichloro-2-ethylamino-pyrimidin-4-yl)-piperazin-1-yl]-ethyl}-cyclohexyl)-acetamide,
*trans*-morpholine-4-carboxylic acid (4-{2-[4-(5,6-dichloro-2-methylamino-pyrimidin-4-yl)-piperazin-1-yl]-ethyl}-cyclohexyl)-amide,
*trans*-3-(4-{2-[4-(5,6-dichloro-2-methylamino-pyrimidin-4-yl)-piperazin-1-yl]-ethyl}-cyclohexyl-1,1-dimethyl-urea,
*trans*-3-(4-{2-[4-(5,6-dichloro-2-ethyl-amino-pyrimidin-4-yl)-piperazin-1-yl]-ethyl}-cyclohexyl}-1,1-dimethyl-urea,
*trans*-1-(4-{2-[4-(5,6-dichloro-2-methylamino-pyrimidin-4-yl)-piperazin-1-yl]-ethyl}-cyclohexyl)-3-ethyl-urea,
*trans*-*N*-(4-{2-[4-(5,6-dichloro-2-methylamino-pyrimidin-4-yl)-piperazin-1-yl]-ethyl}-cyclohexyl-propionamide,
*trans*-N-(4-(2-[4-(2-amino-5,6-dichloro-pyrimidin-4-yl)-piperazin-1-yl]-ethyl)-cyclohexyl)-acetamide,
*trans*-1-(4-{2-[4-(5,6-dichloro-2-methylamino-pyrimidin-4-yl)-piperazin-1-yl]-ethyl}-cyclohexyl)-3-methyl-urea,
*trans*-*N*-(4-{2-[4-(5,6-dichloro-2-methylamino-pyrimidin-4-yl)-piperazin-1-yl]-ethyl}-cyclohexyl)-benzamide,
*trans*-3-bromo-*N*-(4-{2-[4-(5,6-dichloro-2-methylamino-pyrimidin-4-yl)-piperazin-1-yl]-ethyl}-cyclohexyl)-benzamide,
and/or geometric isomers and/or stereoisomers and/or diastereomers and/or salts and/or hydrates and/or solvates thereof.

In another embodiment, the present invention includes compounds of formula (III): 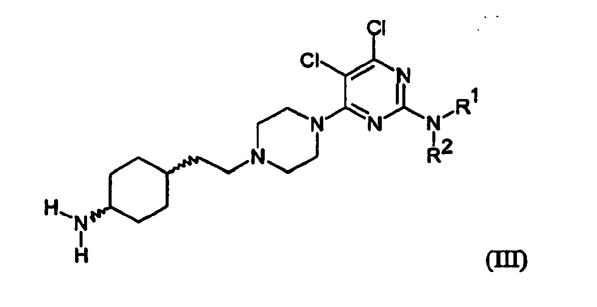 wherein
R¹ represents hydrogen or C₁₋₄ alkyl group, and
R² represents hydrogen or C₁₋₄ alkyl group.

In a further embodiment, the compound of formula (III) is selected from:
*trans*-(4-{4-[2-4-amino-cyclohexyl)ethyl]-piperazin-1-yl}-5,6-dichloro-pyrimidin-2-yl)-methyl-amine,
*trans*-(4-{4-[2-(4-amino-cyclohexyl)-ethyl]-piperazin-1-yl}-5,6-dichloro-pyrimidin-2-yl)-dimethyl-amine,
*trans*-(4-{4-[2-(4-amino-cyclohexyl)-ethyl]-piperazin-1-yl}-5,6-dichloro-pyrimidin-2-yl)-ethyl-amine,
*trans*-(4-{4-[2-(4-amino-cyclohexyl)-ethyl]-piperazin-1-yl}-5,6-dichloro-pyrimidin-2-yl)-amine.

### Synthetic Processes

The present invention also provides processes for preparing compounds of formula (I).

In one embodiment, the present invention is directed to a process (Method A) for preparing compounds of formula (I) wherein
Q represents C₁₋₄ alkyl, NR³R⁴, phenyl, optionally substituted phenyl, 1-pyrrolidinyl, 1-piperidinyl, 4-R⁵-piperazin-1-yl or 4-morpholinyl group,
R¹ represents hydrogen or C₁₋₄ alkyl group,
R² represents hydrogen or C₁₋₄ alkyl group,
R³ represents hydrogen, C₁₋₄ alkyl group, phenyl or optionally substituted phenyl, R⁴ represents hydrogen, C₁₋₄ alkyl group, phenyl or optionally substituted phenyl, and
R⁵ represents hydrogen or C₁₋₄ alkyl group;
said process involving reacting an acid- or carbamoylchoride of formula (II): 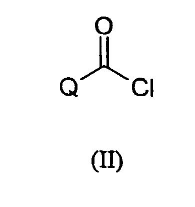 wherein Q is as described above;
with an amine of formula (III): 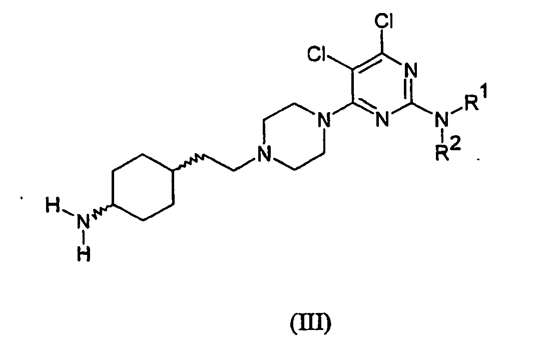 wherein
R¹ represents hydrogen or C₁₋₄ alkyl group, and
R² represents hydrogen or C₁₋₄ alkyl group.

The process of Method A may be carried out by methods known to one of ordinary skill in the art, for example, by suspending or dissolving the appropriate amine of formula (III), or a salt thereof, in a suitable solvent (*e.g*. tetrahydrofuran, dimethylformamide, chlorinated hydrocarbons or hydrocarbons) and adding the appropriate acid- or carbamoylchloride of formula (II) to this suspension or solution, in the presence of a base (*e.g*. triethylamine). The reaction can be carried out advantageously between about -10 °C and about 60 °C. Reaction progress may be monitored by thin layer chromatography. The reaction time is typically about 6-60 h. Work-up of the reaction mixture can be carried out by different known methods. The products can be purified, *e.g*. by crystallization or by column chromatography.

In another embodiment, the present invention is directed to a process (Method B) for preparing compounds of formula (I) wherein
Q represents NR³R⁴;
R¹ represents hydrogen or C₁₋₄ alkyl group;
R² represents hydrogen or C₁₋₄ alkyl group;
R³ represents hydrogen, C₁₋₄ alkyl group, phenyl or optionally substituted phenyl; and
R⁴ represents hydrogen, C₁₋₄ alkyl group, phenyl or optionally substituted phenyl,
said process involving reacting an isocyanate of formula (IV):

R⁶-NCO (IV)

wherein R⁶ represents C₁₋₄ alkyl group, phenyl or optionally substituted phenyl,
with an amine of formula (III): 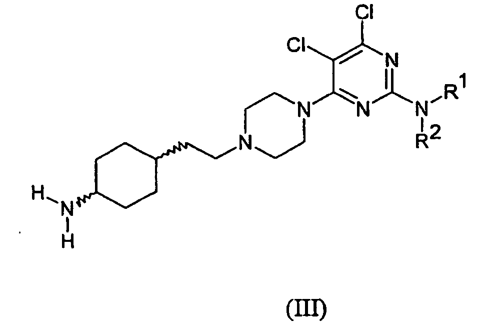 wherein
R¹ represents hydrogen or C₁₋₄ alkyl group,
R² represents hydrogen or C₁₋₄ alkyl group.

The process of Method B may be carried out by methods known to one of ordinary skill in the art, for example, by suspending or dissolving the appropriate amine of formula (III), or a salt thereof, in a suitable solvent (*e.g*. tetrahydrofuran, *N*,*N*-dimethylformamide, chlorinated hydrocarbons or hydrocarbons) and adding the appropriate isocyanate of formula (IV) to this suspension or solution, if necessary, in the presence of a base (*e.g*. triethylamine). The reaction can be carried out advantageously between about 5 °C and about 50 °C. Reaction progress may be monitored by thin layer chromatography. The reaction time is typically about 6-10 h. Work-up of the reaction mixture can be carried out by different known methods. The products can be purified, *e.g*. by crystallization or by column chromatography.

In yet another embodiment, the present invention is directed to a process (Method C) for preparing compounds of formula (I) wherein
Q represents amino
R¹ represents hydrogen or C₁₋₄ alkyl group,
R² represents hydrogen or C₁₋₄ alkyl group
said process involving reacting a cyanate, e.g., potassium cyanate or sodium cyanate with an amine of formula (III) 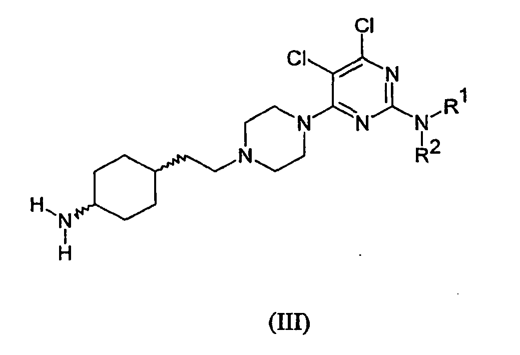 wherein
R¹ represents hydrogen or C₁₋₄ alkyl group,
R² represents hydrogen or C₁₋₄ alkyl group.

The process reaction of Method C may be carried out by methods known to one of ordinary skill in the art, for example, the transformation a compound of formula (III) to a compound of formula (I) may be carried out in an alcoholic solvent (*e.g*. methyl or ethyl alcohol) in the presence of a base (*e.g*. triethylamine), and potassium or sodium cyanate advantageously at reflux temperature. The reaction time is typically about 2-24 hours. Work-up of the reaction mixture can be carried out by different known methods. The products can be purified, *e.g*. by crystallization or by column chromatography.

The acid- or carbamoylchlorides of formula (II) and the isocyanates of formula (IV) are either commercially available or can be synthesized by different methods known to one of ordinary skill in the art. Potassium and sodium cyanate salts are commercially available.

Compounds of formula (III) may be prepared by methods known to one of ordinary skill in the art, *e.g*. by reacting the aldehyde of formula (V): 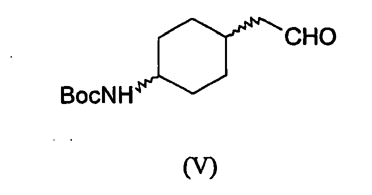 wherein Boc is a tert-butoxycarbonyl group,
with a piperazine of formula (VI): 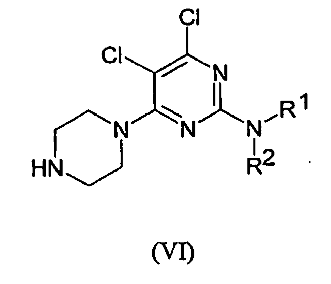 wherein the meaning of R¹ and R² is as described above for formula (III), under reductive amination conditions, followed by removal of the Boc protecting group. The reaction may be carried out in an inert solvent (*e.g*. chlorinated hydrocarbons, alkanols or ethers) in the presence of a reductive agent, for example, sodium borohydride, sodium cyanoborohydride or sodium triacetoxyborohydride. The reaction temperature is between about 0 °C and about room temperature. The reaction time is typically about 2-24 h. Deprotection may be carried out using, e.g., trifluoroacetic acid or hydrochloric acid in a suitable solvent.

Synthesis of aldehydes of formula (V) are described, e.g., in J. Med. Chem. 43, 1878, (2000).

Compounds of formula (VI) may synthesized by methods known to one of ordinary skill in the art, *e.g.* by reacting 1-Boc-piperazine with a pyrimidine of a formula (VII). 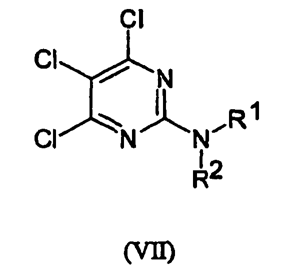 wherein the meaning of R¹ and R² is as described above for formula (III), under alkylation conditions followed by removal of the Boc protecting group. The reaction may be carried out in an inert solvent (*e.g*. chlorinated hydrocarbons, hydrocarbons, acetonitrile, *N*,*N-*dimethylformamide and ketones) in the presence of organic or inorganic base (e. g. triethylamine, sodium or potassium carbonate) advantageously between about 60°C and about 150 °C . The reaction time is about typically 2-24 hours. Work-up of the reaction mixture can be carried out by different known methods. The products can be purified, *e.g*. by crystallization or by column chromatography. Deprotection may be carried out using, e.g., trifluoroacetic acid or hydrochloric acid.

Compounds of a formula (VII) are described, e.g., in J. Med. Chem., 25, 1459, (1982). 1-Boc-piperazine is commercially available.

### Formulations

For use in medicine, the compounds of formula (I) of the present invention and/or geometric isomers and/or stereoisomers and/or diastereomers and/or physiologically acceptable salts and/or hydrates and/or solvates thereof are usually administered as a standard pharmaceutical composition. The present invention therefore provides in a further aspect pharmaceutical compositions comprising a compound of formula (I) and/or geometric isomers and/or stereoisomers and/or diastereomers and/or physiologically acceptable salts and/or hydrates and/or solvates thereof and physiologically acceptable carriers.

The compounds of formula (I) of the present invention and/or geometric isomers and/or stereoisomers and/or diastereomers and/or physiologically acceptable salts and/or hydrates and/or solvates thereof may be administered by any convenient method, for example by oral, parental, buccal, sublingual, nasal, rectal or transdermal administration and the pharmaceutical compositions adapted accordingly.

The compounds of formula (I) of the present invention and/or geometric isomers and/or stereoisomers and/or diastereomers and%or physiologically acceptable salts and/or hydrates and/or solvates thereof which are active when given orally can be formulated as liquids or solids, for example syrups, suspensions or emulsions, tablets, capsules and lozenges.

A liquid formulation of the compounds of formula (I) of the present invention and/or geometric isomers and/or stereoisomers and/or diastereomers and/or physiologically acceptable salts and/or hydrates and/or solvates thereof generally consists of a suspension or solution of the compound of formula (I) and/or geometric isomers and/or stereoisomers and/or diastereomers and/or salts and/or hydrates and/or solvates thereof in a suitable liquid carrier(s), for example an aqueous solvent, such as water, ethanol or glycerol, or a non-aqueous solvent, such as polyethylene glycol or an oil. The formulation may also contain one or more suspending agent, preservative, flavouring or colouring agent, or combinations thereof.

A composition in the solid form of a tablet can be prepared using any suitable pharmaceutical carrier(s) routinely used for preparing solid formulations. Examples of such carriers include magnesium stearate, starch, lactose, sucrose or cellulose

A composition in the solid form of a capsule can be prepared using routine encapsulation procedures. For example, pellets containing the active ingredient can be prepared using standard carriers and then filled into a hard gelatine capsule; alternatively, a dispersion or suspension can be prepared using any suitable pharmaceutical carrier(s), for example aqueous gums, celluloses, silicates or oils and the dispersion or suspension then filled into a soft gelatine capsule.

Parenteral compositions are typically a solution or suspension of the compound of formula (I) of the present invention and/or geometric isomers and/or stereoisomers and/or diastereomers and/or physiologically acceptable salts and/or hydrates and/or solvates thereof in a sterile aqueous carrier or parenterally acceptable oil, for example polyethylene glycol, polyvinyl pyrrolidone, lecithin, arachis oil or sesame oil. Alternatively, the solution can be lyophilised and then reconstituted with a suitable solvent just prior to administration.

Compositions of the present invention for nasal administration containing a compound of formula (I) and/or geometric isomers and/or stereoisomers and/or diastereomers and/or physiologically acceptable salts and/or hydrates and/or solvates thereof may conveniently be formulated as aerosols, drops, gels and powders. Aerosol formulations of the present invention typically comprise a solution or fine suspension of the compound of formula (I) and/or geometric isomers and/or stereoisomers and/or diastereomers and/or physiologically acceptable salts and/or hydrates and/or solvates thereof in a physiologically acceptable aqueous or non-aqueous solvent and are usually presented in a single or multidose quantities in sterile form is a sealed container, which can take the form of a cartridge or refill for use with an atomising device. Alternatively, the sealed container may be a unitary dispensing device, such as a single dose nasal inhaler or an aerosol dispenser fitted with a metering valve which is intended for disposal once the contents of the container have been exhausted. Where the dosage form comprises an aerosol dispenser, it will contain a propellant which can be a compressed gas, such as compressed air or an organic propellant, such as a fluorochlorohydrocarbon. The aerosol dosage form can also take the form of a pump-atomiser. Compositions of the present invention containing a compound of formula (I) and/or geometric isomers and/or stereoisomers and/or diastereomers and/or physiologically acceptable salts and/or hydrates and/or solvates thereof suitable for buccal or sublingual administration include tablets, lozenges and pastilles, wherein the active ingredient is formulated with a carrier, such as sugar and acacia, tragacanth, or gelatine and glycerol

Compositions of the present invention containing a compound of formula (I) and/or geometric isomers and/or stereoisomers and/or diastereomers and/or physiologically acceptable salts and/or hydrates and/or solvates thereof for rectal administration are conveniently in the form of suppositories containing a conventional suppository base, such as cocoa butter.

Compositions of the present invention containing a compound of formula (I) and/or geometric isomers and/or stereoisomers and/or diastereomers and/or physiologically acceptable salts and/or hydrates and/or solvates thereof for transdermal administration include ointments, gels and patches.

The compositions of the present invention containing a compound of formula (I) and/or geometric isomers and/or stereoisomers and/or diastereomers and/or physiologically acceptable salts and/or hydrates and/or solvates thereof are preferably in a unit dose form, such as a tablet, capsule or ampoule.

The following are examples of suitable pharmaceutical formulations of the present invention.
a) Intravenous injection

| | |
|---|---|
| Compound of formula (I) | 1-40 mg |
| Buffer | to pH ca 7 |
| Solvent/complexing agent | to 100 ml |

b) Bolus injection

| | |
|---|---|
| Compound of formula (I) | 1-40 mg |
| Buffer | to pH ca 7 |
| Co-solvent | to 5 ml |

| | |
|---|---|
| Buffer: suitable buffers include citrate, phosphate, sodium hydroxide/hydrochloric acid. Solvent: typically water but may also include cyclodextrins (1-100 mg) and cosolvents, such as propylene glycol, polyethylene glycol and alcohol. | |

c) Tablet

| | |
|---|---|
| Compound of formula (I) | 1-40 mg |
| Diluent/Filter(may also include cyclodextrins) | 50-250 mg |
| Binder | 5-25 mg |
| Disintegrant (may also include cyclodextrins) | 5-50 mg |
| Lubricant | 1-5 mg |
| Cyclodextrin | 1-100 mg |

| | |
|---|---|
| Diluent: *e.g*. microcrystalline cellulose, lactose starch. Binder: *e.g*. polyvinylpyrrolidone, hydroxypropylmethylcellulose. Disintegrant: *e.g*. sodium starch glycolate, crospovidone. Lubricant: *e.g*. magnesium stearate, sodium stearyl fumarate | |

d) Oral suspension

| | |
|---|---|
| Compound of formula (I) | 1-40 mg |
| Suspending agent | 0.1-10 mg |
| Diluent | 20-60 mg |
| Preservative | 0.01-1.0 mg |
| Buffer | to pH ca 5-8 |
| Co-solvent | 0-40 mg |
| Flavour | 0.01-1.0 mg |
| Colourant | 0.001-0.1 mg |

| | |
|---|---|
| Suspending agent: *e.g*. xanthan gum, microcrystalline cellulose. Diluent: *e.g*. sorbitol solution, typically water. Preservative: *e.g*. sodium benzoate. Buffer: *e.g*. citrate. Co-solvent: *e.g*. alcohol, propylene glycol, polyethylene glycol, cyclodextrin. | |

### Medical Use

The compounds of formula (I) of the present invention, in contrast to known antipsychotics, have been found to exhibit very high affinity for dopamine D₃ receptors, high-to-moderate affinity for dopamine D₂ receptors and no affinity for adrenergic alpha-1 receptors. The compounds are expected to be useful in the treatment and/or prevention of disease states in which D₃ and/or D₂ receptors are involved in the disease pathology and thus their modulation is required, or in which modulation of D₃ and/or D₂ receptors exerts beneficial effect on the state and/or process of the disease.

Dysfunction of the dopaminergic neurotransmitter system is involved in the pathology of several neuropsychiatric and neurodegenerative disorders such as schizophrenia, mania, bipolar disorders, drug abuse, dementia, cognitive dysfunctions, and Parkinson's disease. The effects of neurotransmitter dopamine is mediated via at least five distinct dopamine receptors belonging to D1- (*i.e*. D₁ and D₅) or D₂- (*i.e*. D₂, D₃ and D₄) families. D₃ receptors have been shown to have characteristic distribution in the mammalian brain. Namely, they were found in high densities in certain limbic structures such as nucleus accumbens, olfactory tubercle and islands of Calleja. Therefore, preferential targeting of the D₃ receptors may be a promising approach for more selective modulation of certain dopaminergic functions and consequently offers successful therapeutic interventions in several abnormalities such as schizophrenia, emotional or cognitive dysfunctions (see, *e.g*., Sokoloff, P. et al.: Nature 1990, 347:146; Schwartz, J.C. et al.: Clin. Neuropharmacol. 1993, 16:295; Schwartz, J.C. et al.: Brain Res. Rev. 2000, 31:277; Levant, B.: Pharmacol, Rev. 1997, 49:231; Laszy, J. et al.: Psychopharmacol. 2005, 179:567), drug abuse (see, *e.g*., Pilla, C. et al.: Nature 199, 400:371; Heidbreder, C.A. et al.: Brain Res. Rev. 2005, 49:77), Parkinson's disease (see, *e.g*., Levant, B. et al.: CNS Drugs 1999, 12:391; Joyce, J.N.: Pharmacol. Therap. 2001, 90:231; Bézard, E. et al.: Nature Medicine 2003, 9:762) and pain (see, *e.g*., Levant, B. et al.: Neurosci. Lett. 2001, 303:9).

The dopamine D₂ receptors are widely distributed in the brain and are known to be involved in numerous physiological functions and pathological states. D₂ antagonists are, for example, widely used as antipsychotics. However, it is also well known that massive antagonism of the D₂ receptors leads to unwanted side effects, such as extrapyramidal motor symptoms, psychomotor sedation, cognitive blunting and endocrine alterations. These side effects seriously restrict the therapeutic utilization of D₂ antagonist compounds (see, *e.g*, Wong, A.H.C. et al.: Neurosci. Biobehav. Rev. 2003, 27:269.; Stahl, S.M. 2002, Essential Psychopharmacology. Neuroscientific Basis and Practical Applications. 2nd Ed. Cambridge University Press).

Cardiovascular side effects (such as orthostatic hypotension associated with dizziness, tachycardia and sometimes syncope) of the first generation antipsychotics (*e.g*. chlorpromazine, thioridazine, chlorprothixene) and second generation antipsychotics (*e.g*. olanzapine, risperidone) are well documented (see, *e.g.,* Pacher, P. and Kecskeméti, V.: Curr. Pharm. Des. 2004, 10:2463; .Brunton,L., Lazo, J, and Parker, K. (eds) Goodman and Gilman's The Pharmacological Basis of Therapeutics, 11th Edition, Mc Graw Hill, 2005, p.462.; Stahl, S.M. 2002, Essential Psychopharmacology. Neuroscientific Basis and Practical Applications. 2nd Ed. p. 409, Cambridge University Press, 2000; http://www.fda.gov/medwatch/safety/2006/Sep_PIs/RisperdalConsta_PI.pdf; https/www.fda.gov/medwatch/safety/2006/Aug_PIs/Zyprexa_PI.pdf). Side effects of this sort hamper or seriously limit the antipsychotic therapy especially in the initial period. All the above mentioned first and second generation antipsychotics show considerable (*i.e*. nanomolar) affinities to adrenergic alpha-1 receptors and it is a common view that the majority of their cardiovascular side effects are mainly related to their alpha-1 antagonist actions. Thus, the lack of adrenergic alpha-1 activity is a highly desirable feature of a potential antipsychotic compound.

The present invention provides novel compounds of formula (I) and/or geometric isomers and/or stereoisomers and/or diastereomers and/or salts and/or hydrates and/or solvates thereof which have high affinity for dopamine D₃ receptors (Ki values less than 3 nM) and, simultaneously, have high-to-moderate affinity for dopamine D₂ receptors (Ki values of 10 to 50 nM) always in a such combination that the D₃ affinity is 5 to 50-times higher than the D₂ affinity. In addition, compounds of formula (I) have no affinity to adrenergic alpha-1 receptors.

In a further aspect, the present invention provides for the use of a compound of formula (I) and/or geometric isomers and/or stereoisomers and/or diastereomers and/or salts and/or hydrates and/or solvates thereof in treating conditions which require preferential modulation of dopamine D₃ and/or D₂ receptors, such as, but not limited to, psychoses (*e.g*. schizophrenia, schizo-affective disorders), cognitive impairment accompanying schizophrenia, mild-to-moderate cognitive deficits, dementia, psychotic states associated with dementia, psychotic depression, mania, bipolar disorder, paranoid and delusional disorders, dyskinetic disorders such as Parkinson's disease, neuroleptic induced parkinsonism, tardive dyskinesia, eating disorders (*e.g*. bulimia nervosa), attention deficit disorders, hyperactivity disorders, depression and depressive states, anxiety disorders, sexual dysfunctions (*e.g*. erectile dysfunctions), sleep disorders, emesis, aggression, autism, drug (*e.g*. alcohol, cocaine, nicotine, opioids) abuse and pain.

The invention also provides the use of a compound of formula (I) and/or geometric isomers and/or stereoisomers and/or diastereomers and/or salts and/or hydrates and/or solvates thereof in the manufacture of a medicament for the treatment of conditions which require modulation of dopamine receptors, especially dopamine D₃ and/or D₂ receptors.

A preferred use for D₃/D₂ ligands according to the present invention is in the treatment of schizophrenia, schizo-affective disorders, cognitive impairment accompanying schizophrenia, mild-to-moderate cognitive deficits, dementia, psychotic states associated with dementia, psychotic depression, mania, bipolar disorder, paranoid and delusional disorders, dyskinetic disorders such as Parkinson's disease, neuroleptic induced parkinsonism, depression and depressive states, anxiety disorders, and drug abuse (*e.g*. cocaine abuse).

The particular combination of the two receptor-actions described above allows the simultaneous manifestation of the beneficial actions of D₃ functional antagonism (*e.g*. cognitive enhancer effect, inhibition of extrapyramidal motor symptoms, inhibitory action on drug abuse) and that of the D₂ functional antagonism (*e.g*. antipsychotic effect). Furthermore, the same combination surprisingly results in cancelling out the disadvantageous features of D₂ antagonism (*e.g*. extrapyramidal symptoms, psychomotor sedation, cognitive disturbances).

### EXAMPLES

The present invention will now be further described by way of the following examples.

The structure of all intermediates and end products were elucidated by IR, NMR and MS spectroscopy.

### Example 1

### 4-(5,6-dichloro-2-methylamino-pyrimidin4-yl)-piperazine-1-carboxyclic acid tert-butyl ester (intermediate compound 1a)

Methyl-(4,5,6-trichloro-pyrimidin-2-yl)-amine (2.8 g, 12.2 mmol), 1-Boc-piperazine (2.27 g, 12.2 mmol), potassium carbonate (0.84 g 6.1 mmol) in water (2.5 ml) and methyl ethyl ketone (50 ml) were refluxed for 12 hours. After cooling to room temperature the precipitate was filtered, and washed with water to give the title compound (2.6 g, 59 %), melting point: 205-206 °C.

Applying the above procedure the following compounds were prepared:
4-(5,6-dichloro-2-dimethylamino-pyrimidn-4-yl)-piperazine-1-carboxylic acid tert-butyl ester, melting point: 129-131°C (intermediate compound 1b);
4-(5,6-dichloro-2-ethylamino-pyrimidin-4-yl)-piperazine-1-carboxylic acid tert-butyl ester, melting point: 164-167 °C (intermediate compound 1c);
4-(2-amino-5,6-dichloro-pyrimidin-4-yl)-piperazine-1-carboxylic acid tert-butyl ester, melting point: 170-175 °C (intermediate compound 1d).

### Example 2

### (4,5-Dichloro-6-piperazin-1-yl-pyrimidin-2-yl)-methyl-amine dihydrochloride (intermediate compound 2a)

2.6 g (7.2 mmol) 4-(5,6-dichloro-2-methylamino-pyrimidin-4-yl)-piperazine-1-carboxylic acid tert-butyl ester was deprotected at 10 °C using 100 ml ethylacetate saturated with gaseous hydrochloric acid. After 4 hours the precipitate was filtered giving the title compound (2.4 g, 100 %), melting at 204-209 °C.

Applying the above procedure the following compounds were prepared:
(4,5-dichloro-6-piperazin-1-yl-pyrimidin-2-yl)-dimethyl-amine dihydrochloride, melting point: 178-184 °C (intermediate compound 2b);
(4,5-dichloro-6-piperazin-1-yl-pyrimidin-2-yl)-ethyl-amine dihydrochloride, melting point: 200-202 °C (intermediate compound 2c);
(4,5-dichloro-6-piperazin-1-yl-pyrimidin-2-yl)-amine dihydrochloride, melting point: 183-185 °C (intermediate compound 2d).

### Example 3

### Trans-(4-{2-[4-(5,6-dichloro-2-methylamino-pyrimidin-4-yl)-piperazin-1-yl]-ethyl}-cyclohexyl)-carbamic acid tert-butyl ester (intermediate compound 3 a)

5.36 g (16 mmol) of (4,5-dichloro-6-piperazin-1-yl-pyrimidin-2-yl)-methyl-amine dihydrochloride and 3.86 g (16 mmol) of *tans*-4-(2-oxoethyl)cyclohexyl-carbamic acid tert-butyl ester were dissolved in dichloromethane (320 ml). 6.7 ml (48 mmol) triethylamine was added, then 5.1 g (24 mmol) sodium triacetoxyborohydride was added portions wise and the reaction mixture was stirred for 20 hours at ambient temperature. 20 % potassium carbonate solution in water (100 ml) was then added. The organic layer was separated, dried and evaporated to dryness *in vacuo.* The residue was triturated with diethyl ether to give the title compound (6.9 g, 88.5 %), melting point: 199-202 °C.

Applying the above procedure the following compounds were prepared:
*trans*-(4-{2-[4-(5,6-dichloro-2-dimethylamino-pyrimidin-4-yl)-piperazin-1-yl]-ethyl}-cyclohexyl)-carbamic acid tert-butyl ester melting point: 169-171 °C (intermediate compound 3b);
*trans*-(4-{2-[4-(5,6-dichloro-2-ethylamino-pyrimidin-4-yl)-piperazin-1-yl]-ethyl}-cyclohexyl)-carbamic acid tert-butyl ester melting point: 164-168 °C (intermediate compound 3c);
*trans*-(4-{2-[4-(2-amino-5,6-dichloro-pyrimidin-4-yl)-piperazin-1-yl]-ethyl}-cyclohexyl)-carbamic acid tert-butyl ester, melting point: 197-199 °C (intermediate compound 3d).

### Example 4

### Trans-(4-{4-[2-(4-amino-cyclohexyl)-ethyl]-piperazin-1-yl}-5,6-dichloro-pyrimidin-2-yl)-methyl-amine trihydrochloride (intermediate compound 4a)

4.88 g (10 mmol) *trans*-(4-{2-[4-(5,6-dichloro-2-methylamino-pyrimidin-4-yl)-piperazin-1-yl]-ethyl}-cyclohexyl)-carbamic acid tert-butyl ester was deprotected at 10 °C using 100 ml ethylacetate saturated with gaseous hydrochloric acid. After 4 hours the precipitate was filtered giving the title compound (4.9 g, 99 %), melting at 325-326 °C.

Applying the above procedure the following compounds were prepared:
*trans*-(4-{4-[2-(4-amino-cyclohexyl)-ethyl]-piperazin-1-yl}-5,6-dichloro-pyrimidin-2-yl)-dimethyl-amine trihydrochloride, melting point: 329-330 °C (intermediate compound 4b)
*trans*-(4-{4-[2-(4-amino-cyclohexyl)-ethyl]-piperazin-1-yl}-5,6-dichloro-pyrimidin-2-yl)-ethyl-amine trihydrochloride, melting point: 318-319 °C (intermediate compound 4c)
*trans*-(4-{4-[2-(4-amino-cyclohexyl)-ethyl]-piperazin-1-yl}-5,6-dichloro-pyrimidin-2-yl)-amine trihydrochloride, melting point: 324-326 °C (intermediate compound 4d)

### Method A

### Tran-N-(4-{2-[4-(5,6-dichloro-2-methylamino-pyrimidin-4-yl)-piperazin-1-yl]-ethyl}-cyclohexyl)-acetamide (Compound 1)

2.28 g (4.6 mmol) *trans*-(4-{4-[2-(4-amino-cyclohexyl)-ethyl]-piperazin-1-yl}-5,6-dichloro-pyrimidin-2-yl)-methyl-amine trihydrochloride was suspended in dichloromethane (50 ml). Triethylamine (3.5 ml, 25.3 mmol) was added followed by the addition of acetyl chloride (0.49 ml, 6.9 mmol). The reaction mixture was stirred for 24 hours at room temperature. The precipitate was filtered, washed with water and purified using column chromatography to give the title compound (1.39 g, 70 %), MS (EI): 430.2 (MH⁺); ¹H NMR (300 MHz, DMSO-d₆ (TMS) + 1 drop of cc. DCl, δ (ppm)): 0.89-0.96 m (2H); 1.06-1.35 m (3H); 1.55-1.82 m (6H); 1.79 s (3H); 2.76 s (3H); 2.98-3.18 m (4H); 3.37-3.58 m (5H); 4.16-4.29 m (2H); 7.90 br (residual NH); 11.35 br (residual NH).

### Method B

### Trans-1-(4-{2-[4-(5,6-dichloro-2-methylamino-pyrimidin-4-yl)-piperazin-1-yl]-ethyl}-cyclohexyl)-3-ethyl-urea (Compound 10)

0.25 g (0.5 mmol) *trans*-(4-{4-[2-(4-amino-cyclohexyl)-ethyl)-piperazin-1-yl}-5,6-dichloro-pyrimidin-2-yl)-methyl-amine trihydrochloride was dissolved in dry dichloromethane (10 ml). Triethylamine (0.28 ml, 2 mmol) was added followed by the addition of ethylisocyanate (0.06 ml, 0.753 mmol), and the reaction mixture was stirred at room temperature for 4 hours. The solvent was removed *in vacuo.* The residue was triturated with water, and the precipitate was filtered to give the title compound (0.17 g, 72 %) MS (EI): 459.2 (MH⁺). ¹H NMR (300 MHz, DMSO-d₆ (TMS) + 1 drop of cc. DCl, δ (ppm)): 0.96 t (3H); 0.88-1.13 m (4H); 1.13-1.31 m (1H); 1.54-1.85 m (6H); 2.74 s (3H); 2.98 q (2H); 2.89-3.16 m (4H); 3.19-3.33 m (1H); 3.34-3.58 m (4H); 4.12-4.30 m (2H).

### Method C

### Trans-(4-{2-[4-(5,6-dichloro-2-methylamino-pyrimidin-4-yl)-piperazin-1-yl]-ethyl}-cyclohexyl)-urea (Compound 2)

0.6 g (1.3 mmol) *trans*-(4-{4-[2-(4-amino-cyclohexyl)-ethyl]-piperazin-1-yl}-5,6-dichloro-pyrimidin-2-yl)-methyl-amine trihydrochlorid was suspended in methanol. Triethylamine (0.36 ml, 2.6 mmol) was added followed by the addition potassium cyanate (0.26 g, 3.12 mmol). The mixture was refluxed for 10 hours. The solvent was removed *in vacuo.* The residue was triturated with water, and the precipitate was filtered to give the title compound (0.42 g 75 %) MS (EI): 431.2 (MH⁺). ¹H NMR (300 MHz, DMSO-d₆ (TMS)+ 1 drop of cc. DCl, δ (ppm)): 0.91-1.12 m (2H); 1.15-1.36 m (3H); 1.56-1.92 m (6H); 2.76 s (3H); 3.00-3.21 m (4H); 3.30-3.61 m (5H); 4.15-4.31 m (2H).

Applying one of the above methods, using the appropriate reactants, the following compounds were prepared:
*trans*-morpholine-4-carboxylic acid (4-{2-[4-(5,6-dichloro-2-ethylamino-pyrimidin-4-yl)-piperazin-1-yl]-ethyl}-cyclohexyl)-amide (Compound 3), MS (EI): 515.2 (MH⁺); ¹H NMR (300 MHz, DMSO-d₆ (TMS), δ (ppm)): 0.84-1.03 m (2H); 1.08 t (3H); 1.12-1.27 m (3H); 1.27-1.40 m (2H); 1.66-1.83 m (4H); 2.24-2.51 m (6H); 3.16-3.27 m (6H); 3.35-3.42 m (1H); 3.43-3.59 m (8H); 6.14 d (1H); 7.40 br. (1H);
*trans*-(4-{2-[4-(5,6-dichloro-2-ethylamino-pyrimidin-4-yl)-piperazin-1-yl]-ethyl}-cyclohexyl)-urea (Compound 4), MS (EI): 445.2 (MH⁺); ¹H NMR (300 MHz, DMSO-d₆ (TMS) + 1drop of cc. TFA, δ (ppm)): 0.86-1.18 m (4H); 1.10 s (3H); 1.15-1.32 m (1H); 1.49-1.61 m (2H); 1.65-1.89 m (4H); 3.03-3.37 m (9H); 3.49-3.62 m (2H); 4.18-4.36 m (2H);
*trans*-*N*-(4-{2-[4-(5,6-dichloro-2-dimethylamino-pyrimidin-4-yl)-piperazin-1-yl]-ethyl}-cyclohexyl)-acetamide (Compound 5), MS (EI): 444.3 (MH⁺); ¹H NMR (300 MHz, DMSO-d₆+ 1drop of cc. TFA (TMS), δ (ppm)): 0.91-1.34 m (5H); 1.45-1.62 m (2H); 1.66-1.85 m (4H); 1.77 s (3H); 3.07 s (6H); 3.07-3.20 m (4H); 3.20-3.36 m (2H); 3.37-3.62 m (3H); 4.22-4.36 m (2H); 7.71 d (1H); 9.77 br (due to protonation);
*trans*-*N*-(4-{2-[4-(5,6-dichloro-2-ethylamino-pyrimidin-4-yl)-piperazin-1-yl]-ethyl}-cyclohexyl)-acetamide (Compound 6), MS (EI): 444.2 (MH⁺); ¹H NMR (300 MHz, CDCl₃ (TMS), δ (ppm)): 1.04-1.14 m (4H); 1.19 t (3H); 1.23-1.30 m (1H); 1.37-1.48 m (2H); 1.77-1.82 m (2H); 1.95 s (3H); 1.94-2.04 m (2H); 2.34-2.43 m (2H); 2.48-2.55 m (4H); 3.30-3.43 m (2H); 3.58-3.77 m (5H); 4.83 t (1H); 5.23 d (1H);
*trans*-morpholine-4-carboxylic acid (4-{2-[4-(5,6-dichloro-2-methylamino-pyrimidin-4-yl)-piperazin-1-yl]-ethyl}-cyclohexyl)-amide (Compound 7), MS (EI): 501.2 (MH⁺); ¹H NMR (500 MHz, DMSO-d₆+DCl (TMS), δ (ppm)): 0.91-1.04 m (2H); 1.12-1.30 m (3H); 1.55-1.66 m (2H); 1.67-1.84 m (4H); 2.76 s (3H); 3.01-3.15 m (4H); 3.19-3.28 m (4H); 3.34-3.43 m (1H); 3.42-3.63 m (8H); 4.03-4.37 m (2H); 7.45 br (residual NH); 11.22 br (residual NH);
*trans*-3-(4-{2-[4-(5,6-dichloro-2-methylamino-pyrimidin-4-yl)-piperazin-1-yl]-ethyl}-cyclohexyl-1,1-dimethyl-urea (Compound 8), MS (EI): 459.2 (MH⁺); ¹H NMR (500 MHz, DMSO-d₆ (TMS) +1 drop of cc. TFA, δ (ppm)): 0.91-1.06 m (2H); 1.14-1.31 m (3H); 1.49-1.64 m (2H); 1.67-1.84 m (4H); 2.77 s (9H); 3.03-3.21 m (4H); 3.21-3.43 m (3H); 3.51-3.62 m (2H); 4.14-4.44 m (2H); 5.49 br. (due to protonation); 7.50 br (1H); 9.83 br (1H);
*trans*-3-(4-{2-[4-(5,6-dichloro-2-ethylamino-pyrimidin-4-yl)-piperazin-1-yl]-ethyl}-cyclohexyl}-1,1-dimethyl-urea (Compound 9), MS (EI): 473.2 (MH⁺); ¹H NMR (500 MHz, DMSO-d₆(TMS)+ 1drop of cc.TFA, δ (ppm)): 0.91-1.03 m (2H); 1.09 t (3H); 1.13-1.28 m (3H); 1.50-1.60 m (2H); 1.66-1.84 m (4H); 2.75 s (6H); 3.00-3.19 m (4H); 3.19-3.39 m (5H); 3.48-3.62 m (2H); 4.07-4.36 m (2H); 5.86 br (due to protonation); 7.53 br (1H); 9.91 br (1H);
*trans*-*N*-(4-{2-[4-(5,6-dichloro-2-methylamino-pyrimidin-4-yl)-piperazin-1-yl]-ethyl}-cyclohexyl-propionamide (Compound 11), MS(EI): 444.2 (MH⁺) ¹H NMR (500 MHz, DMSO-d₆ (TMS) 1drop of cc. TFA, δ (ppm)): 0.97 t (3H); 0.95-1.05 m (2H); 1.06-1.18 m (2H); 1.18-1.29 m (1H); 1.51-1.59 m (2H); 1.68-1.81 m (4H); 2.03 q (2H); 2.76 s (3H); 3.03-3.34 m (6H); 3.40-3.51 m (1H); 3.51-3.60 m (2H); 4.13-4.39 m (2H); 7.42, 7.52 br. (due to protonation); 7.60 d (1H); 9.72 br. (1H);
*trans*-N-(4-{2-[4-(2-amino-5,6-dichloro-pyrimidin-4-yl)-piperazin-1-yl]-ethyl}-cyclohexyl)-acetamide (Compound 12), MS (EI): 416.2 (MH⁺); ¹H NMR (300 MHz, DMSO-d₆ (TMS), δ (ppm)): 0.83-1.28 m (5H); 1.28-1.40 m (2H); 1.65-1.82 m (4H); 1.76 s (3H); 2.26-2.35 m (2H); 2.37-2.48 m (4H); 3.35-3.54 m (5H); 7.65 d (1H); 6.83 s (2H);
*trans*-1-(4-{2-[4-(5,6-dichloro-2-methylamino-pyrimidin-4-yl)-piperazin-1-yl]-ethyl} - cyclohexyl)-3-methyl-urea (Compound 13), MS (EI): 445,3 (MH⁺); ¹H NMR (400 MHz, DMSO-d₆ (TMS)+ 1 drop of cc. DCl, δ (ppm)): 0.90-1.17 m (4H); 1.19-1.31 m (1H); 1.58-1.84 m (6H); 2.54 s (3H); 2.76 s (3H); 3.02-3.16 m (4H); 3.22-3.33 m (1H); 3.42-3.57 m (4H); 4.10-4.30 m (2H); 7.44 br (residual NH); 11.33 br (residual NH)
*trans-N-*(4-{2-[4-(5,6-dichloro-2-methylamino-pyrimidin-4-yl)-piperazin-1-yl]-ethyl}-cyclohexyl)-benzamide (Compound 14), MS(EI): 492.4 (MH⁺); ¹H NMR (400 MHz, DMSO-d₆ (TMS)+ 1 drop of cc. DCl, δ (ppm)): 0.98-1.11 m (2H); 1.21-1.44 m (3H); 1.61-1.70 m (2H); 1.73-1.90 m (4H); 2.76 s (3H); 2.97-3.19 m (4H); 3.42-3.56 m (4H); 3.66-3.83 m (1H); 4.09-4.32 m (2H); 7.41-7.54 m (3H); 7.82-7.87 m (2H); 8.26 br (residual NH); 11.33 br (residual NH);
*trans*-3-bromo-*N*-(4-{2-[4-(5,6-dichloro-2-methylamino-pyrimidin-4-yl)-piperazin-1-yl]-ethyl}-cyclohexyl)-benzamide (Compound 15), MS(EI): 571.3 (MH⁺); ¹H NMR (400 MHz, CDCl₃ + MeOD-d₄ (TMS), δ (ppm)): 1.06-1.42 m (5H); 1.48-1.65 m (2H); 1.79-1.88 m (2H); 2.04-2.13 m (2H); 2.43-2.85 m (6H); 2.91 s (3H); 3.62-3.99 m (5H); 7.29-7.32 m (1H); 7.59-7.64 m (1H); 7.66-7.71 m (1H); 7.87-7.90 m (1H).

### Biological Test Methods

### 1. D₃ receptor binding

Binding assays were carried out on rat recombinant D₃ receptors (Perkin-Elmer, Cat. No. 6110139) expressed in Sf9 cells using [³H]spiperone (0.44-1.49 nM) as ligand and haloperidol (10 µM) for determination of non-specific binding. The assay was performed according to the supplier's assay protocol (Cat.No.: 3110139).

### 2. D₂ Receptor Binding

D₂ receptor binding was determined as described by Creese et al. (Eur. J. Pharmacol., 60:55-66, 1979) on rat brain striatal membrane preparation using [³H]spiperone (0.4-1.3 nM) as ligand. Non-specific binding was determined in the presence of 1 µM (+) butaclamol.

### 3. Alpha-1 Receptor Binding

Alpha-1 receptor binding studies were performed according to the methods described by Greengrass and Bremner (Eur. J. Pharmacol., 55:323-326, 1979) on rat cortical membrane preparation using [³H]-prazosine (0.22-0.37 nM) as ligand. The non-specific binding was determined in the presence of 10 µM phentolamine.

### 4. Amphetamine-Induced Hypermotility

One hour after the oral administration of doses of the test compound or vehicle, male Wistar rats were subcutaneously treated with d-amphetamine (0.5 mg/kg, sc.) and were individually placed in activity cages for one hour. Locomotor activity was measured in a four-channel activity monitor equipped with infrared photobeams Horizontal movement was determined as the number of beam interruptions. Mean ± SE of horizontal activity data of each group was calculated. Percent inhibition of amphetamine-induced increase in locomotion was calculated for each dose of the tested compound. The ED-50 value was determined by linear regression fitted to the dose-response plot.

### 5. Catalepsy Test

Thirty minutes after the oral treatment with the test compounds male Wistar rats weighing 200-220 g (n=10/group) were placed in extra-ordinary position: placing both forepaws of the rat on a 10 cm high podium. Animals were considered to be cataleptic if they did not correct their body posture within 30 sec. The frequency of cataleptic animals was determined at one, two, three, four and five hours after the treatment. Minimum effective (cataleptic) dose was defined as the dose causing catalepsy at least at two readings (*i.e*. either at two time points in the same animal or in two different animals at any of the time points).

### 6. Scopolamine-Induced Learning Disturbance in the Water-Labyrinth

The learning process of rats was assessed in a 3-choice point water-labyrinth system. The number of directional turning errors was recorded in three daily trials for three experimental days. Male Wistar rats weighing 180-200 g (n=10 per groups) were treated orally with vehicle or the test compounds 1 hour before each daily session. Scopolamine (3 mg/kg ip.) as amnestic agent was injected 30 minutes prior to the first daily trial. Mean ± SE of errors committed in all the trials was calculated in each group. Percent inhibition of scopolamine-induced increase in the number of errors was calculated for each dose of the tested compound.

Dopamine D₃ and D₂ and adrenergic alpha-1 receptor binding data of selected compounds of the present invention are listed in Table 1. Ki (nM) data are given.

**Table 1.**

| Compound | D₃ | D₂ | | α-1 | |
|---|---|---|---|---|---|
| | Ki(nM) | Ki(nM) | Sel. | Ki(nM) | Sel. |
| 1 | < 1 | 15-50 | 22 | >>1000 | n.a. |
| 2 | 1-3 | 15-50 | 27 | >>1000 | n.a. |
| 6 | 1-3 | 15-50 | 23 | >>1000 | n.a. |
| 7 | 1-3 | 5-15 | 7 | >>1000 | n.a. |
| 8 | 1-3 | 15-50 | 7 | >>1000 | n.a. |
| 10 | <1 | 5-15 | 17 | >>1000 | n.a. |
| 11 | <1 | 5-15 | 43 | >>1000 | n.a. |
| Olanzapine | 76 | 96 | 1.3 | 25.1 | 0.33 |
| Risperidone | 13 | 13 | 1.0 | 0.88 | 0.07 |

| | | | | | |
|---|---|---|---|---|---|
| n.a.: not applicable, due to the lack of alpha-1 binding Sel. = D₂/D₃ selectivity, i.e., Ki for D₂ receptor divided by Ki for D₃ receptor | | | | | |

The most prominent side effects of the first generation antipsychotic compounds (*e.g*. chlorpromazine and haloperidol) and at higher doses even those of second generation (atypical) antipsychotics (*e.g*. risperidone) are the extrapyramidal symptoms such as pseudo-parkinsonism and tardive dyskinesia and the orthostatic hypotension. The former two are the result of massive blockade of D₂ receptors in the basal ganglia whereas the latter is the consequence of antagonism of alpha-1 receptors.

As can be seen from Table 1, the compounds of the present invention are very highly potent ligands at D₃ receptors (Ki values are less than 3 nM) and moderately potent ligands at dopamine D₂ receptors (Ki values between 5 and 50 nM) showing 5 to 50 fold selectivity for D₃ over D₂ receptors. Coupling the very high D₃ affinity to the moderate D₂ affinity in this particular proportion allows the beneficial (*e.g*. antipsychotic) actions of a D₂ antagonist to be preserved, while at the same time, impeding (by the D₃ effects) the appearance of the disadvantageous consequences of massive D₂ receptor blockade, such as extrapyramidal symptoms or cognitive disturbances. It is therefore anticipated that no or greatly diminished adverse effects related to D₂ receptors will occur in the course of therapeutical application of compounds of the present invention. Furthermore, as well as favourably modulating the dopamine D₂ receptor-mediated functions, action of the compounds of formula (I) of the present invention on dopamine D₃ receptors will also result in additional therapeutically beneficial effects *e.g*. cognitive improvement, diminution of negative and depressive symptoms. In addition, the compounds have no affinity to adrenergic alpha-1 receptors (Ki values are higher than 1000 nM for each compound) and thus have extremely high D₃/alpha-1 selectivity. From the lack of affinity of the compounds to adrenergic alpha-1 receptors the lack of cardiovascular side effects (*e.g*. orthostatic hypotension and associated symptoms such as dizziness, tachycardia) is anticipated.

The beneficial effects of the compounds of formula (I) of the present invention carrying the above described particular combination of D₃ and D₂ receptor binding affinities were demonstrated *in vivo,* in methods used to measure antipsychotic effect (amphetamine hypermotility), cognitive enhancer activity (scopolamine-induced learning disturbance) and extrapyramidal side-effect (catalepsy test).The results are shown in Tables 2 and 3.

**Table 2**

| **Effects of compounds of formula (I) on amphetamine-induced hypermotility and in the catalepsy test** | | | |
|---|---|---|---|
| compound | Inhibition of amphetamine-induced hypermotility (ED50, mg/kg) | Catalepsy (MED, mg/kg) | TI^{a} |
| 1 | 0.14 | 100 | 714 |
| 8 | 0.14 | >25 | >179 |
| Olanzapine | 1.8 | 40 | 22 |
| Risperidone | 0.15 | 6.0 | 40 |

| | | | |
|---|---|---|---|
| a: therapeutic index - catalepsy MED divided by amphetamine hypermotility ED50 | | | |

As can be seen from Table 2, compounds of formula (I) of the present invention have highly potent antipsychotic activity (inhibition of amphetamine-induced hypermotility) as can be predicted from their high to moderate dopamine D₂ receptor affinities. With regard to cataleptogenic (*i.e*. extrapyramidal side effect-inducing) potential, compounds of formula (I) of the present invention are highly superior to the reference drugs olanzapine and risperidone both in absolute (MED) and relative (TI) terms. Since olanzapine and risperidone show equal binding affinity to the D₃ and D₂ dopamine receptors (see Table 1) whereas compounds of formula (I) of the present invention preferably bind to the D₃ receptor (their D₃ affinity is 5 to 50 fold higher than the D₂ affinity) in such a way that they have high or very high affinity to dopamine D₃ receptors and moderate to high affinity to dopamine D₂ receptors, the results of Table 2 also demonstrate that such particular combination of D₃ and D₂ affinities indeed result in preservation of the beneficial antipsychotic action with simultaneous elimination of the disadvantageous extrapyramidal side-effect (catalepsy).

**Table 3**

| **Effects of compounds of formula (I) on scopolamine-induced learning disturbance** | | |
|---|---|---|
| Compound | Dose (mg/kg) | % inhibition^{a} |
| 1 | 0.1 | 36 |
| | 0.2 | 58 |
| | 0.4 | 61 |
| | 0.8 | 44 |
| 8 | 0.025 | 38 |
| | 0.05 | 65 |
| | 1 | 31 |
| Olanzapine | 1 | *-12* |
| | 3 | *-49* |
| Risperidone | 0.5 | *-15* |

| | | |
|---|---|---|
| a: negative values mean further *impairment* in learning performance | | |

Data in Table 3 show that compounds of formula (I) of the present invention do exert cognitive enhancing effect (as evidenced by the considerable inhibition of the learning disrupting effect of scopolamine) in contrast to olanzapine and risperidone which further impaired rather than improved the learning performance of scopolamine treated animals. These findings demonstrate the beneficial effect of the very high to high D₃ receptor affinity characteristic for the compounds of formula (I) of the present invention and also point to the importance of the particular 5 to 50-fold D₃/D₂ selectivity ratio possessed by these compounds. In the case of risperidone and olanzapine, compounds which showed equal affinity to the D₃ and D₂ receptors, the deleterious effect of D₂ antagonism on learning overcame the beneficial cognitive action of D₃ antagonism while in case of compounds of formula (I) of the present invention the 5 to 50-fold higher D₃ receptor affinity cancelled out the disadvantageous effect (cognitive disturbance in this case) of D₂ antagonism.

## Claims

1. A compound of formula (I): 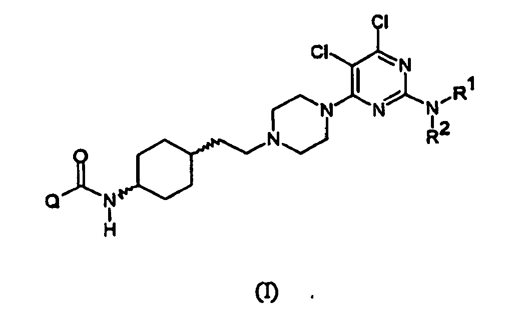 wherein
Q represents C₁₋₄ alkyl, NR³R⁴, phenyl, optionally substituted phenyl, 1-pyrrolidinyl, 1-piperidinyl, 4-R⁵-piperazin-1-yl or 4-morpholinyl group,
R¹represents hydrogen or C₁₋₄ alkyl group;
R² represents hydrogen or C₁₋₄ alkyl group;
R³ represents hydrogen, C₁₋₄ alkyl group, phenyl or optionally substituted phenyl;
R⁴ represents hydrogen, C₁₋₄ alkyl group, phenyl or optionally substituted phenyl;
R⁵ represents hydrogen or C₁₋₄ alkyl group;
and/or geometric isomers and/or stereoisomers and/or diastereomers and/or salts and/or hydrates and/or solvates thereof, wherein the term "optionally substituted phenyl" means a phenyl group which can be substituted in any position by one or more halogen, C₁₋₄ alkyl, C₁₋₄ alkoxy, trifluoromethyl and/or cyano group, or combinations thereof.

2. A compound of claim 1, wherein
Q represents C₁₋₄ alkyl, NR³R⁴ or 4-morpholinyl group,
R¹ represents hydrogen atom or C₁₋₄ alkyl group;
R² represents hydrogen atom or C₁₋₄ alkyl group;
R³ represents hydrogen atom or C₁₋₄ alkyl group; and
R⁴ represents hydrogen atom or C₁₋₄ alkyl group.

3. The compound of formula (I) according to claim 1 which is selected from
*trans*-N-(4-{2-[4-(5,6-dichloro-2-methylamino-pyrimidin4-yl)-piperazin-1-yl]-ethyl]-cyclohexyl}-acetamide,
*trans*-(4-{2-[4-(5,6-dichloro-2-methylamino-pyrimidin-4-yl)-piperazin-1-yl]-ethyl}-cyclohexyl)-urea,
*trans*-morpholine4-carboxylic acid (4-{2-[4-(5,6-dichloro-2-ethylamino-pyrimidin-4-yl)-piperazin-1-yl]-ethyl}-cyclohexyl)-amide,
*trans-*(4-{2-[4-(5,6-dichloro-2-ethylamino-Pyrimidin-4-yl)-piperazin-1-yl]-ethyl}-cyclohexyl)-urea,
*trans*-*N*-(4-{2-[4-(5,6-dichloro-2-dimethylamino-pyrimidin-4-yl)-piperazin-1-yl]-ethyl}-cyclohexyl)-acetamide,
*trans-N*-(4-{2-[4-(5,6-dichloro-2-ethylamino-pyrimidin-4-yl)-piperazin-1-yl]-ethyl}-cyclohexyl)-acetamide,
*trans*-morpholine-4-carboxylic acid (4-{2-[4-(5,6-dichloro-2-methylamino-pyrimidin-4-yl)-piperazin-1-yl]-ethyl}-cyclohexyl)-amide,
*trans*-3-(4-{2-[4-(5,6-dichloro-2-methylamino-pyrimidin-4-yl)-piperazin-1-yl]-ethyl}-cyclohexyl-1,1-dimethyl-urea,
*trans*-3-(4-{2-[4-(5,6-dichloro-2-ethylamino-pyrimidin-4-yl)-piperazin-1-yl]-ethyl}-cyclohexyl}-1,1-dimethyl-urea,
*trans*-1-(4-{2-[4-(5,6-dichloro-2-methylamino-pyrimidin-4-yl)-piperazin-1-yl]-ethyl}-cyclohexyl)-3-ethyl-urea,
*trans*-*N*-(4-{2-[4-(5,6-dichloro-2-methylamino-pyrimidin-4-yl)-piperazin-1-yl]-ethyl}-cyclohexyl-propionamide,
*trans*-N-(4-{2-[4-(2-amino-5,6-dichloro-pyrimidin-4-yl)-piperazin-1-yl]-ethyl}-cyclohexyl)-acetamide,
*trans*-1-(4-{2-[4-(5,6-dichloro-2-methylamino-pyrimidin4-yl)-piperazin-1-yl]-ethyl}-cyclohexyl)-3-methyl-urea,
*trans*-*N*-(4-{2-[4-(5,6-dichloro-2-methylamino-pyrimidin-4-yl)-piperezin-1-yl]-ethyl}-cyclohexyl)-benzamide,
*trans*-3-bromo-*N*-(4-{2-[4-(5,6-dichloro-2-methylamino-pyrimidin-4-yl)-piperazin-1-yl]-ethyl}-cyclohexyl)-benzamide,
and/or geometric isomers and/or stereoisomers and/or diastereomers and/or salts and/or hydrates and/or solvates thereof.

4. A compound of formula (III): 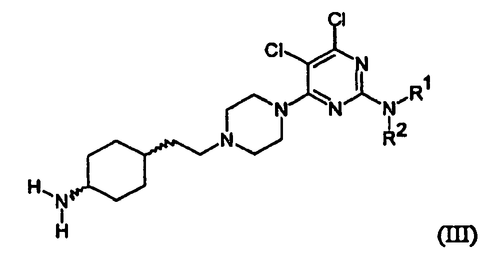 wherein
R¹ represents hydrogen or C₁₋₄ alkyl group, and
R² represents hydrogen or C₁₋₄ alkyl group.

5. A process for preparing a compound of formula (I): 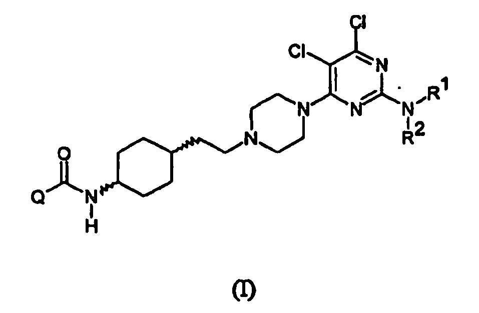 and/or geometric isomers and/or stereoisomers and/or diastereomers and/or salts and/or hydrates and/or solvates thereof,
wherein
Q represents C₁₋₄ alkyl, -NR³R⁴, phenyl, optionally substituted phenyl, 1-pyrrolidinyl, 1-piperidinyl, 4-R⁵-piperazin-1-yl or 4-morpholinyl group,
R¹ represents hydrogen or C₁₋₄ alkyl group,
R² represents hydrogen or C₁₋₄ alkyl group,
R³ represents hydrogen, C₁₋₄ alkyl group, phenyl, or optionally substituted phenyl, R⁴ represents hydrogen, C₁₋₄ alkyl group, phenyl or optionally substituted phenyl, R⁵ represents hydrogen or C₁₋₄ alkyl group
wherein the term "optionally substituted phenyl" means a phenyl group which can be substituted in any position by one or more halogen, C₁₋₄ alkyl, C₁₋₄ alkoxy, trifluoromethyl and/or cyano group, or combinations thereof **comprising reacting an acid- or carbamoylchoride of formula (II):** 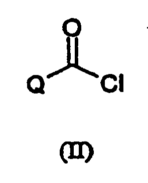 wherein Q is as described above;
with an amino of formula (III): 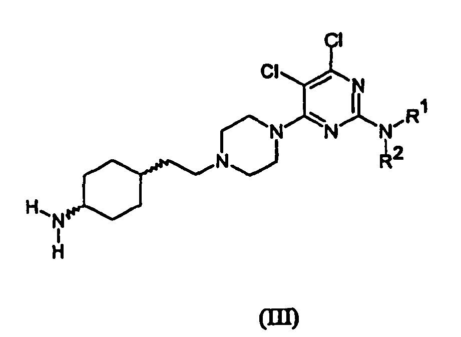 wherein
R¹ represents hydrogen or C₁₋₄ alkyl group,
R² represents hydrogen or C₁₋₄ alkyl group,
and, where appropriate, separating the enantiomers and/or diastereomers, and/or cis- and/or trans-isomers of compounds of formula (I), or intermediates thereto, by conventional methods,
and optionally thereafter forming salts and/or hydrates and/or solvates of the compound of formula (I).

6. A process for preparing a compound of formula (I): 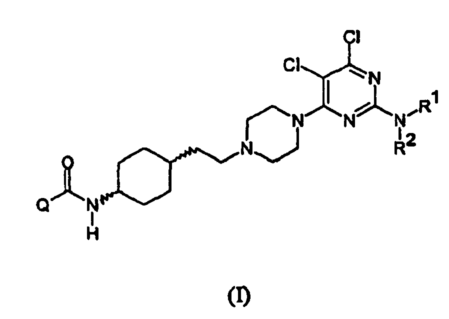 and/or geometric isomers and/or stereoisomers and/or diastereomers and/or salts and/or hydrates and/or solvates thereof,
wherein
Q represents NR³R⁴,
R¹ represents hydrogen or C₁₋₄ alkyl group,
R² represents hydrogen or C₁₋₄ alkyl group,
R³ represents hydrogen, C₁₋₄ alkyl group, phenyl or optionally substituted phenyl,
R⁴ represents hydrogen, C₁₋₄ alkyl group, phenyl or optionally substituted phenyl, wherein the term "optionally substituted phenyl" means a phenyl group which can be substituted in any position by one or more halogen, C₁₋₄ alkyl, C₁₋₄ alkoxy, trifluoromethyl and/or cyano group, or combinations thereof
comprising reacting an isocyanate of formula (IV):
R⁶-NCO (IV)
wherein R⁶ represents C₁₋₄ alkyl group, phenyl or optionally substituted phenyl group
with an amine of formula (III): 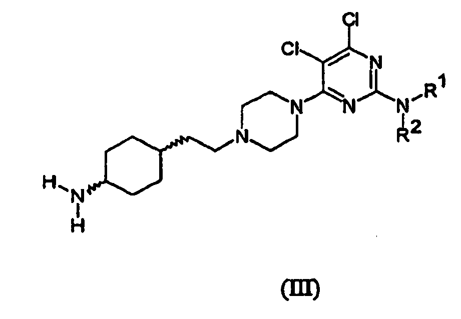 wherein
R¹ represents hydrogen atom or C₁₋₄ alkyl group,
R² represents hydrogen atom or C₁₋₄ alkyl group,
and, where appropriate, separating the enantiomers and/or diastereomers, and/or cis- and/or trans-isomers of compounds of formula (I), or intermediates thereto, by conventional methods,
and optionally thereafter forming salts and/or hydrates and/or solvates of the compound of formula (I).

7. A process for preparing a compound of formula (I): 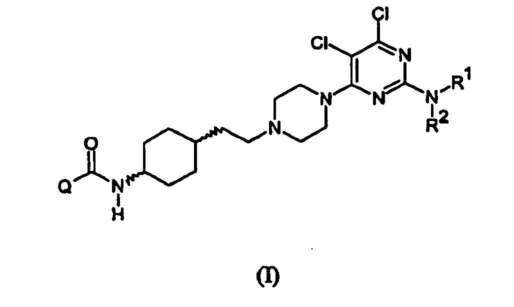 and/or geometric isomers and/or stereoisomers and/or diastereomers and/or salts and/or hydrates and/or solvates thereof,
wherein
Q represents amino,
R¹ represents hydrogen or C₁₋₄ alkyl group,
R² represents hydrogen or C₁₋₄ alkyl group, comprising
comprising reacting potassium cyanate or sodium cyanate with an amine of formula (III) 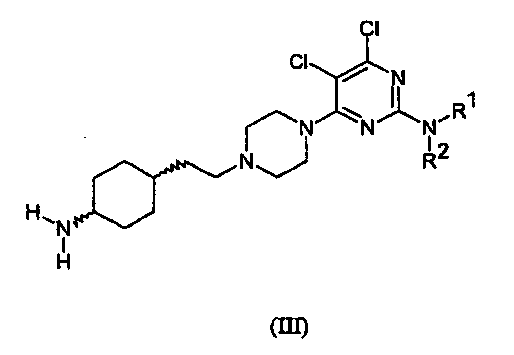 wherein
R¹ represents hydrogen or C₁₋₄ alkyl group;
R² represents hydrogen atom or C₁₋₄ alkyl group;
and, where appropriate, separating the enantiomers and/or diiastereomers, and/or cis- and/or trans- isomers of compounds of formula (I), or intermediates thereto, by conventional methods,
and optionally thereafter forming salts and/or hydrates and/or solvates of the compound of formula (I).

8. A pharmaceutical composition comprising a compound of formula (I) according to claim 1 and one or more physiologically acceptable carrier(s).

9. The use of a compound of formula (I) according to claim 1 in the manufacture of a medicament for the treatment and/or prevention of psychoses, drug abuse, cognitive impairment accompanying schizophrenia, mild-to-moderate cognitive deficits, dementia, psychotic states associated with dementia, eating disorders, attention deficit disorders, hyperactivity disorders, psychotic depression, mania, bipolar disorder, paranoid and delusional disorders, dyskinetic disorders, depression and depressive states, anxiety disorders, sexual dysfunctions, sleep disorders, emesis, aggression, autism or pain.

10. The use according to claim 9,
wherein the psychosis is schizophrenia or a schizo-affective disorder; the drug abuse is alcohol, cocaine, nicotine or opioid abuse; the eating disorder is bulimia nervosa; the dyskinetic disorder is Parkinson's disease, neuroleptic induced parkinsonism or tardive dyskinesia; the sexual dysfunctions is erectile dysfunctions.

11. A compound of formula (I) according to claim 1 for use in treating and/or preventing psychoses, drug abuse, cognitive impairment accompanying schizophrenia, mild-to-moderate cognitive deficits, dementia, psychotic states associated with dementia, eating disorders, attention deficit disorders, hyperactivity disorders, psychotic depression, mania, bipolar disorder, paranoid and delusional disorders, dyskinetic disorders, depression and depressive states, anxiety disorders, sexual dysfunctions, sleep disorders, emesis, aggression, autism or pain.

12. The compound of claim 11,
wherein the psychosis is schizophrenia or a schizo-affective disorder; the drug abuse is alcohol, cocaine, nicotine or opioid abuse; the eating disorder is bulimia nervosa; the dyskinetic disorder is Parkinson's disease, neuroleptic induced parkinsonism or tardive dyskinesia; the sexual dysfunctions is erectile dysfunctions.

## Patentansprüche

1. Verbindung der Formel (I): 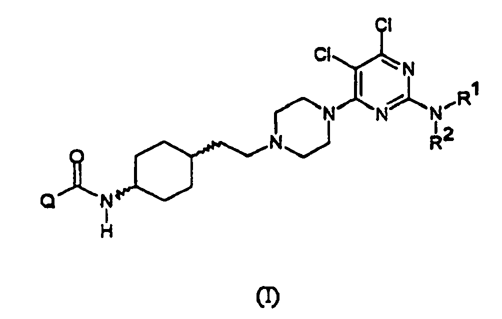 worin
Q C₁₋₄-Alkyl, -NR³R⁴, Phenyl, optional substituiertes Phenyl, 1-Pyrrolidinyl, 1-Piperidinyl, 4-R⁵-Piperazin-1-yl oder 4-Morpholinylgruppe darstellt,
R¹ Wasserstoff oder C₁₋₄-Alkylgruppe darstellt;
R² Wasserstoff oder C₁₋₄-Alkylgruppe darstellt;
R³ Wasserstoff, C₁₋₄-Alkylgruppe, Phenyl oder optional substituiertes Phenyl darstellt;
R⁴ Wasserstoff, C₁₋₄-Alkylgruppe, Phenyl oder optional substituiertes Phenyl darstellt;
R⁵ Wasserstoff oder C₁₋₄-Alkylgruppe darstellt;
und/oder geometrische Isomere und/oder Steroisomere und/oder Diasteromere und/oder Salze und/oder Hydrate und/oder Solvate davon, worin der Begriff "optional substituiertes Phenyl" eine Phenylgruppe bezeichnet, die in beliebigen Positionen mit einem oder mehreren Halogen-, C₁₋₄-Alkyl-, C₁₋₄-Alkoxy-, Trifluoromethyl- und/oder Cyanogruppen oder Kombinationen davon substituiert sein kann.

2. Verbindung gemäß Anspruch 1, worin
Q C₁₋₄-Alkyl-, -NR³R⁴- oder 4-Morpholinylgruppe darstellt,
R¹ Wasserstoffatom oder C₁₋₄-Alkylgruppe darstellt;
R² Wasserstoffatom oder C₁₋₄-Alkylgruppe darstellt;
R³ Wasserstoffatom oder C₁₋₄-Alkylgruppe darstellt; und
R⁴ Wasserstoffatom oder C₁₋₄-Alkylgruppe darstellt.

3. Verbindung der Formel (I) gemäß Anspruch 1, die ausgewählt ist aus
trans-N-(4-{2-[4-(5,6-Dichloro-2-methylaminopyrimidin-4-yl)-piperazin-1-yl]-ethyl}-cyclohexyl)-acetamid,
trans-(4-{2-[4-(5,6-Dichloro-2-methylaminopyrimidin-4-yl)-piperazin-1-yl]-ethyl}-cyclohexyl)-harnstoff,
trans-Morpholin-4-carbonsäure-(4-{2-[4-(5,6-dichloro-2-ethylaminopyrimidin-4-yl)-piperazin-1-yl]-ethyl]-cyclohexyl}-amid,
trans-(4-{2-[4-(5,6-Dichloro-2-ethylaminopyrimidin-4-yl)-piperazin-1-yl]-ethyl}-cyclohexyl)-harnstoff, trans-N-(4-{2-[4-(5,6-Dichloro-2-dimethylaminopyrimidin-4-yl)-piperazin-1-yl]-ethyl}-cyclohexyl)-acetamid,
trans-N-(4-{2-[4-(5,6-Dichloro-2-ethylaminopyrimidin-4-yl)-piperazin-1-yl]-ethyl}-cyclohexyl)-acetamid,
trans-Morpholin-4-carbonsäure-(4-{2-[4-(5,6-dichloro-2-methylaminopyrimidin-4-yl)-piperazin-1-yl]-ethyl}-cyclohexyl)-amid,
trans-3-(4-{2-[4-(5,6-Dichloro-2-methylaminopyrimidin-4-yl)-piperazin-1-yl]-ethyl}-cyclohexyl)-1,1-dimethylharnstoff,
trans-3-(4-{2-[4-(5,6-Dichloro-2-ethylaminopyrimidin-4-yl)-piperazin-1-yl]-ethyl}-cyclohexyl)-1,1-dimethylharnstoff,
trans-1-(4-{2-[4-(5,6-Dichloro-2-methylaminopyrimidin-4-yl)-piperazin-1-yl]-ethyl}-cyclohexyl)-3-ethylharnstoff,
trans-N-(4-{2-[4-(5,6-Dichloro-2-methylaminopyrimidin-4-yl)-piperazin-1-yl]-ethyl}-cyclohexyl)-propionamid,
trans-N-(4-{2-[4-(2-Amino-5,6-dichloropyrimidin-4-yl)-piperazin-1-yl]-ethyl}-cyclohexyl)-acetamid,
trans-1-(4-{2-[4-(5,6-Dichloro-2-methylaminopyrimidin-4-yl)-piperazin-1-yl]-ethyl}-cyclohexyl)-3-methylharnstoff,
trans-N-(4-{2-[4-(5,6-Dichloro-2-methylaminopyrimidin-4-yl)-piperazin-1-yl]-ethyl}-cyclohexyl)-benzamid,
trans-3-Bromo-N-(4-{2-[4-(5,6-dichloro-2-methylaminopyrimidin-4-yl)-piperazin-1-yl]-ethyl}-cyclohexyl)-benzamid,
und/oder geometrische Isomere und/oder Steroisomere und/oder Diastereomere und/oder Salze und/oder Hydrate und/oder Solvate davon.

4. Verbindung der Formel (III) 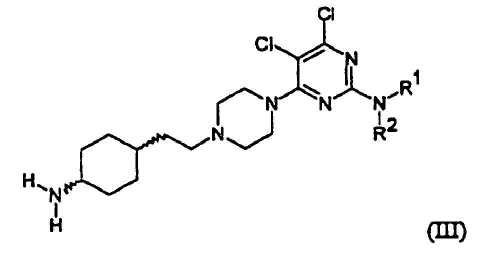 worin
R¹ Wasserstoff oder C₁₋₄-Alkylgruppe darstellt; und
R² Wasserstoff oder C₁₋₄-Alkylgruppe darstellt.

5. Verfahren zur Herstellung einer Verbindung der Formel (I): 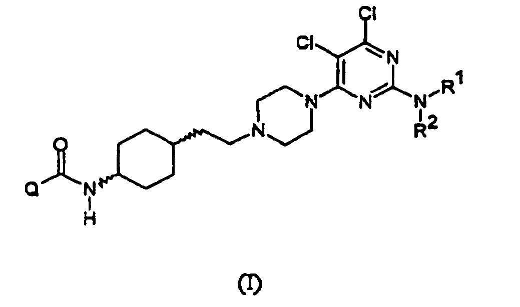 und/oder geometrische Isomeren und/oder Steroisomeren und/oder Diasteromeren und/oder Salzen und/oder Hydraten und/oder Solvaten davon,
worin
Q C₁₋₄-Alkyl, -NR³R⁴, Phenyl, optional substituiertes Phenyl, 1-Pyrrolidinyl, 1-Piperidinyl, 4-R⁵-Piperazin-1-yl oder 4-Morpholinylgruppe darstellt,
R¹ Wasserstoff oder C₁₋₄-Alkylgruppe darstellt;
R² Wasserstoff oder C₁₋₄-Alkylgruppe darstellt;
R³ Wasserstoff, C₁₋₄-Alkylgruppe, Phenyl oder optional substituiertes Phenyl darstellt;
R⁴ Wasserstoff, C₁₋₄-Alkylgruppe, Phenyl oder optional substituiertes Phenyl darstellt;
R⁵ Wasserstoff oder C₁₋₄-Alkylgruppe darstellt;
worin der Ausdruck "optional substituiertes Phenyl" eine Phenylgruppe bezeichnet, die in beliebiger Stellung durch ein oder mehrere Halogen, C₁₋₄-Alkyl, C₁₋₄-Alkoxy, Trifluoromethyl und/oder Cyanogruppen oder Kombinationen davon substituiert sein kann,
umfassend das Umsetzen eines Säure- oder Carbamoylchlorids der Formel (II): 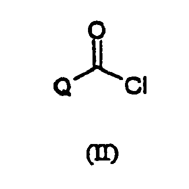 worin Q ist, wie oben beschrieben; mit einem Amin der Formel (III) 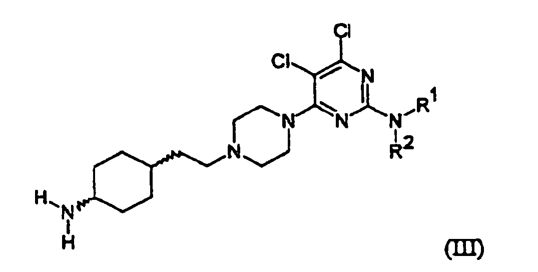 worin
R¹ Wasserstoff oder C₁₋₄-Alkylgruppe darstellt,
R² Wasserstoff oder C₁₋₄-Alkylgruppe darstellt,
und, wenn erforderlich, das Trennen der Enantionmere und/oder Diasteromere und/oder cis- und/oder trans-Isomere der Verbindungen der Formel (I) oder deren Intermediate durch herkömmliche Verfahren,
und optional anschließendes Bilden von Salzen und/oder Hydraten und/oder Solvaten der Verbindung der Formel (I) .

6. Verfahren zur Herstellung einer Verbindung der Formel (I): 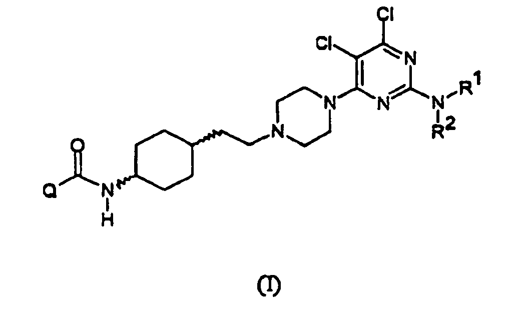 und/oder geometrische Isomeren und/oder Steroisomeren und/oder Diasteromeren und/oder Salzen und/oder Hydraten und/oder Solvaten davon,
worin
Q NR³R⁴ darstellt,
R¹ Wasserstoff oder C₁₋₄-Alkylgruppe darstellt,
R² Wasserstoff oder C₁₋₄-Alkylgruppe darstellt,
R³ Wasserstoff, C₁₋₄-Alkylgruppe, Phenyl oder optional substituiertes Phenyl darstellt,
R⁴ Wasserstoff, C₁₋₄-Alkylgruppe, Phenyl oder optional substituiertes Phenyl darstellt,
worin der Ausdruck "optional substituiertes Phenyl" eine Phenylgruppe bezeichnet, die in beliebiger Stellung durch ein oder mehrere Halogen, C₁₋₄-Alkyl, C₁₋₄-Alkoxy, Trifluoromethyl und/oder Cyanogruppen oder Kombinationen davon substituiert sein kann,
umfassend das Umsetzen eines Isocyanats der Formel (IV):
R⁶-NCO
worin R⁶ C₁₋₄-Alkylgruppe, Phenyl oder optional substituierte Phenylgruppe darstellt
mit einem Amin der Formel (III) 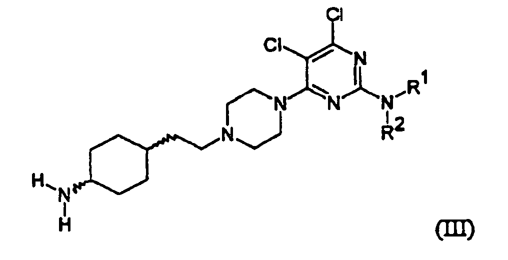 worin
R¹ Wasserstoff oder C₁₋₄-Alkylgruppe darstellt,
R² Wasserstoff oder C₁₋₄-Alkylgruppe darstellt
und, wenn erforderlich, das Trennen der Enantionmere und/oder Diasteromere und/oder cis- und/oder trans-Isomere der Verbindungen der Formel (I) oder deren Intermediate durch herkömmliche Verfahren,
und optional anschließendes Bilden von Salzen und/oder Hydraten und/oder Solvaten der Verbindung der Formel (I) .

7. Verfahren zur Herstellung einer Verbindung der Formel (I) : 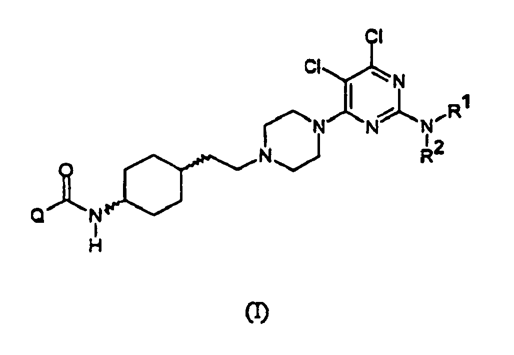 und/oder geometrische Isomeren und/oder Steroisomeren und/oder Diasteromeren und/oder Salzen und/oder Hydraten und/oder Solvaten davon,
worin
Q Amino darstellt,
R¹ Wasserstoff oder C₁₋₄-Alkylgruppe darstellt,
R² Wasserstoff oder C₁₋₄-Alkylgruppe darstellt, umfassend das Umsetzen von Kaliumcyanat oder Natriumcyanat mit einem Amin der Formel (III) 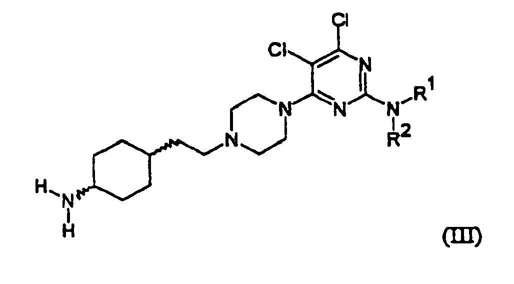 worin
R¹ Wasserstoff oder C₁₋₄-Alkylgruppe darstellt,
R² Wasserstoff oder C₁₋₄-Alkylgruppe darstellt
und, wenn erforderlich, das Trennen der Enantionmere und/oder Diasteromere und/oder cis- und/oder trans-Isomere der Verbindungen der Formel (I) oder deren Intermediate durch herkömmliche Verfahren,
und optional anschließendes Bilden von Salzen und/oder Hydraten und/oder Solvaten der Verbindung der Formel (I) .

8. Pharmazeutische Zusammensetzung, umfassend eine Verbindung der Formel (I) gemäß Anspruch 1 und einen oder mehrere physiologisch annehmbare Träger.

9. Verwendung einer Verbindung der Formel (I) gemäß Anspruch 1, in der Herstellung eines Medikaments zur Behandlung und/oder Prävention von Psychosen, Drogenmissbrauch, Schizophrenie-begleitende kognitive Störung, milde bis moderate kognitive Defizite, Demenz, mit Demenz assoziierte psychotische Zustände, Essstörungen, Aufmerksamkeitsdefizitstörungen, Hyperaktivitätsstörungen, psychotische Depression, Manie, bipolare Störung, paranoide und wahnhafte Störungen, dyskinetische Störungen, Depression und depressive Zustände, Angststörungen, sexuelle Dysfunktionen, Schlafstörungen, Emesis, Aggression, Autismus oder Schmerz.

10. Verwendung gemäß Anspruch 9, worin die Psychose Schizophrenie oder eine Schizo-affektive Störung ist; der Drogenmissbrauch Alkohol-, Kokain-, Nikotin- oder Opioidmissbrauch ist; die Essstörung Bulimia nervosa ist; die dyskinetische Störung Parkinsonsche Krankheit, neuroleptisch induzierter Parkinsonismus oder tarditive Dyskinesie ist; die sexuelle Dysfunktion erektile Dysfunktion ist.

11. Verbindung der Formel (I) gemäß Anspruch 1 zur Verwendung im Behandeln und/oder Verhindern von Psychosen, Drogenmissbrauch, Schizophrenie-begleitende kognitive Störung, milde bis moderate kognitive Defizite, Demenz, mit Demenz assoziierte psychotische Zustände, Essstörungen, Aufmerksamkeitsdefizitstörungen, Hyperaktivitätsstörungen, psychotische Depression, Manie, bipolare Störung, paranoide und wahnhafte Störungen, dyskinetische Störungen, Depression und depressive Zustände, Angststörungen, sexuelle Dysfunktionen, Schlafstörungen, Emesis, Aggression, Autismus oder Schmerz.

12. Verbindung gemäß Anspruch 11, worin die Psychose Schizophrenie oder eine Schizo-affektive Störung ist; der Drogenmissbrauch Alkohol-, Kokain-, Nikotin- oder Opioidmissbrauch ist; die Essstörung Bulimia nervosa ist; die dyskinetische Störung Parkinsonsche Krankheit, neuroleptisch induzierter Parkinsonismus oder tarditive Dyskinesie ist; die sexuelle Dysfunktion erektile Dysfunktion ist.

## Revendications

1. Composé de formule (I) : 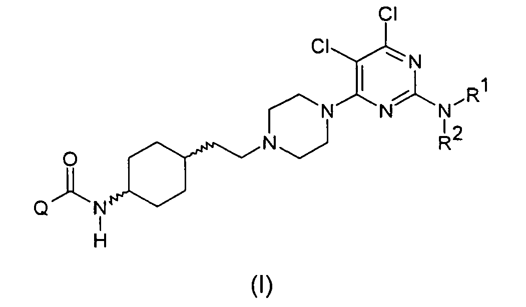 dans laquelle
Q représente un groupe alkyle en C₁ à C₄, -NR³R⁴ phényle, phényle éventuellement substitué, 1-pyrrolidinyle, 1-pipéridinyle, 4-R⁵-pipérazin-1-yle ou 4-morpholinyle,
R¹ représente l'hydrogène ou un groupe alkyle en C₁ à C₄ ;
R² représente l'hydrogène ou un groupe alkyle en C₁ à C₄ ;
R³ représente l'hydrogène, un groupe alkyle en C₁ à C₄, phényle ou phényle éventuellement substitué ;
R⁴ représente l'hydrogène, un groupe alkyle en C₁ à C₄, phényle ou phényle éventuellement substitué ;
R⁵ représente l'hydrogène ou un groupe alkyle en C₁ à C₄ ;
et/ou ses isomères géométriques et/ou stéréoisomères et/ou diastéréomères et/ou sels et/ou hydrates et/ou solvates, l'expression "phényle éventuellement substitué" signifiant un groupe phényle qui peut être substitué en n'importe quelle position par un ou plusieurs halogènes ou groupes alkyle en C₁ à C₄, alcoxy en C₁ à C₄, trifluorométhyle et/ou cyano, ou leurs combinaisons.

2. Composé selon la revendication 1, dans lequel
Q représente un groupe alkyle en C₁ à C₄, -NR³R⁴ ou 4-morpholinyle,
R¹ représente un atome d'hydrogène ou un groupe alkyle en C₁ à C₄ ;
R² représente un atome d'hydrogène ou un groupe alkyle en C₁ à C₄ ;
R³ représente un atome d'hydrogène ou un groupe alkyle en C₁ à C₄ ; et
R⁴ représente un atome d'hydrogène ou un groupe alkyle en C₁ à C₄.

3. Composé de formule (I) selon la revendication 1, qui est choisi parmi les suivants :
*trans*-N-(4-{2-[4-(5,6-dichloro-2-méthylamino-pyrimidin-4-yl)-pipérazin-1-yl]-éthyl]-cyclohexyl}-acétamide,
*trans*-(4-{2-[4-(5,6-dichloro-2-méthylamino-pyrimidin-4-yl)-pipérazin-1-yl]-éthyl}-cyclohexyl)-urée,
(4-{2-[4-(5,6-dichloro-2-éthylamino-pyrimidin-4-yl)-pipérazin-1-yl]-éthyl}-cyclohexyl)-amide d'acide *trans*-morpholine-4-carboxylique,
*trans*-(4-{2-[4-(5,6-dichloro-2-éthylamino-pyrimidin-4-yl)-pipérazin-1-yl]-éthyl}-cyclohexyl)-urée,
*trans-N*-(4-{2-[4-(5,6-dichloro-2-diméthylamino-pyrimidin-4-yl)-pipérazin-1-yl]-éthyl}-cyclohexyl)-acétamide,
*trans-N*-(4-{2-[4-(5,6-dichloro-2-éthylamino-pyrimidin-4-yl)-pipérazin-1-yl]-éthyl}-cyclohexyl)-acétamide,
(4-{2-[4-(5,6-dichloro-2-méthylamino-pyrimidin-4-yl)-pipérazin-1-yl]-éthyl}-cyclohexyl)-amide d'acide *trans*-morpholine-4-carboxylique,
*trans*-3-(4-{2-[4-(5,6-dichloro-2-méthylamino-pyrimidin-4-yl)-pipérazin-1-yl]-éthyl}-cyclohexyl-1,1-diméthyl-urée,
*trans*-3-(4-{2-[4-(5,6-dichloro-2-éthyl-amino-pyrimidin-4-yl)-pipérazin-1-yl]-éthyl}-cyclohexyl}-1,1-diméthyl-urée,
*trans*-1-(4-{2-[4-(5,6-dichloro-2-méthylamino-pyrimidin-4-yl)-pipérazin-1-yl]-éthyl}-cyclohexyl)-3-éthyl-urée,
*trans*-*N*-(4-{2-[4-(5,6-dichloro-2-méthylamino-pyrimidin-4-yl)-pipérazin-1-yl]-éthyl}-cyclohexyl-propionamide,
*trans*-N-(4-{2-[4-(2-amino-5,6-dichloro-pyrimidin-4-yl)-pipérazin-1-yl]-éthyl}-cyclohexyl)-acétamide,
*trans*-1-(4-{2-[4-(5,6-dichloro-2-méthylamino-pyrimidin-4-yl)-pipérazin-1-yl]-éthyl}-cyclohexyl)-3-méthyl-urée,
*trans*-*N*-(4-{2-[4-(5,6-dichloro-2-méthylamino-pyrimidin-4-yl)-pipérazin-1-yl]-éthyl}-cyclohexyl)-benzamide,
*trans*-3-bromo-*N*-(4-{2-[4-(5,6-dichloro-2-méthylamino-pyrimidin-4-yl)-pipérazin-1-yl]-éthyl}-cyclohexyl)-benzamide,
et/ou leurs isomères géométriques et/ou stéréoisomères et/ou diastéréomères et/ou sels et/ou hydrates et/ou solvates.

4. Composé de formule (III) : 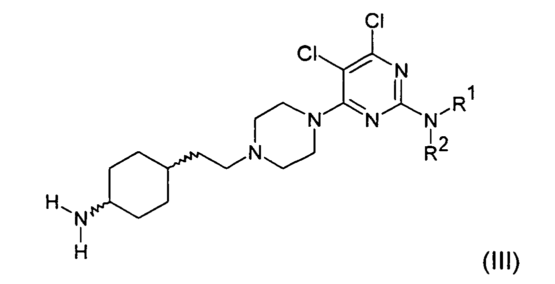 dans laquelle
R¹ représente l'hydrogène ou un groupe alkyle en C₁ à C₄, et
R² représente l'hydrogène ou un groupe alkyle en C₁ à C₄.

5. Procédé pour préparer un composé de formule (I) : 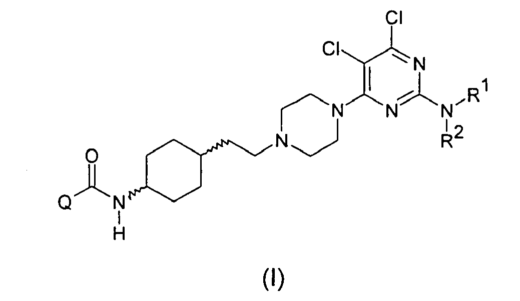 et/ou ses isomères géométriques et/ou stéréoisomères et/ou diastéréomères et/ou sels et/ou hydrates et/ou solvates,
formule dans laquelle
Q représente un groupe alkyle en C₁ à C₄, -NR³R⁴, phényle, phényle éventuellement substitué, 1-pyrrolidinyle, 1-pipéridinyle, 4-R⁵-pipérazin-1-yle ou 4-morpholinyle,
R¹ représente l'hydrogène ou un groupe alkyle en C₁ à C₄,
R² représente l'hydrogène ou un groupe alkyle en C₁ à C₄,
R³ représente l'hydrogène, un groupe alkyle en C₁ à C₄, phényle ou phényle éventuellement substitué,
R⁴ représente l'hydrogène, un groupe alkyle en C₁ à C₄, phényle ou phényle éventuellement substitué,
R⁵ représente l'hydrogène ou un groupe alkyle en C₁ à C₄ l'expression "phényle éventuellement substitué" signifiant un groupe phényle qui peut être substitué en n'importe quelle position par un ou plusieurs halogènes ou groupes alkyle en C₁ à C₄, alcoxy en C₁ à C₄, trifluorométhyle et/ou cyano, ou leurs combinaisons,
comprenant la réaction d'un chlorure d'acide ou de carbamoyle de formule (II) : 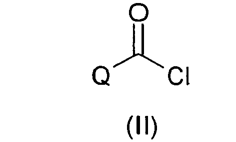 dans laquelle Q est tel que décrit ci-dessus ;
avec une amine de formule (III) : 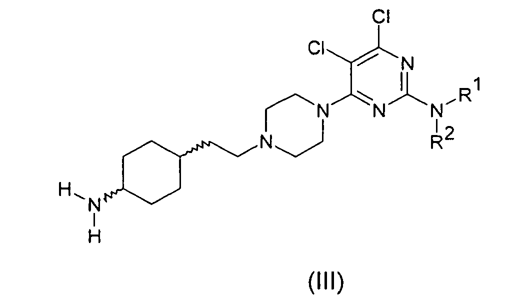 dans laquelle
R¹ représente l'hydrogène ou un groupe alkyle en C₁ à C₄,
R² représente l'hydrogène ou un groupe alkyle en C₁ à C₄, et, lorsque c'est approprié, la séparation des énantiomères et/ou diastéréomères, et/ou des isomères cis et/ou trans de composés de formule (I), ou d'intermédiaires pour ceux-ci, par des procédés conventionnels,
et éventuellement ensuite la formation de sels et/ou hydrates et/ou solvates du composé de formule (I).

6. Procédé pour préparer un composé de formule (I) : 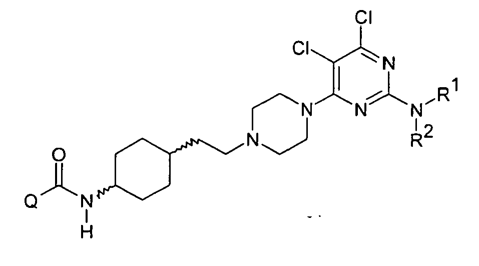 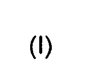 et/ou ses isomères géométriques et/ou stéréoisomères et/ou diastéréomères et/ou sels et/ou hydrates et/ou solvates,
formule dans laquelle
Q représente NR³R⁴,
R¹ représente l'hydrogène ou un groupe alkyle en C₁ à C₄,
R² représente l'hydrogène ou un groupe alkyle en C₁ à C₄,
R³ représente l'hydrogène, un groupe alkyle en C₁ à C₄, phényle ou phényle éventuellement substitué,
R⁴ représente l'hydrogène, un groupe alkyle en C₁ à C₄, phényle ou phényle éventuellement substitué,
l'expression "phényle éventuellement substitué" signifiant un groupe phényle qui peut être substitué en n'importe quelle position par un ou plusieurs halogènes ou groupes alkyle en C₁ à C₄, alcoxy en C₁ à C₄, trifluorométhyle et/ou cyano, ou leurs combinaisons,
comprenant la réaction d'un isocyanate de formule (IV) :
R⁶-NCO (IV)
dans laquelle R⁶ représente un groupe alkyle en C₁ à C₄, phényle ou phényle éventuellement substitué,
avec une amine de formule (III) : 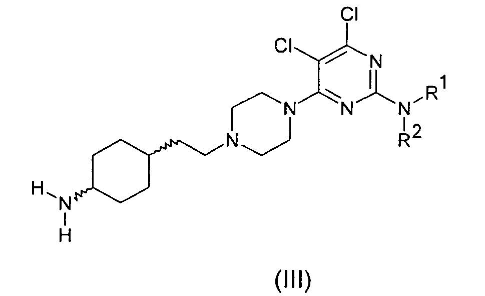 dans laquelle
R¹ représente un atome d'hydrogène ou un groupe alkyle en C₁ à C₄,
R² représente un atome d'hydrogène ou un groupe alkyle en C₁ à C₄,
et, lorsque c'est approprié, la séparation des énantiomères et/ou diastéréomères, et/ou des isomères cis et/ou trans de composés de formule (I), ou d'intermédiaires pour ceux-ci, par des procédés conventionnels,
et éventuellement ensuite la formation de sels et/ou hydrates et/ou solvates du composé de formule (I).

7. Procédé pour préparer un composé de formule (I) : 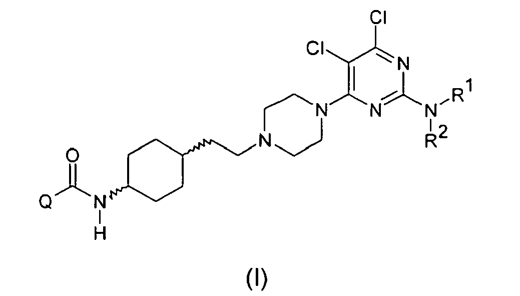 et/ou ses isomères géométriques et/ou stéréoisomères et/ou diastéréomères et/ou sels et/ou hydrates et/ou solvates,
formule dans laquelle
Q représente amino,
R¹ représente l'hydrogène ou un groupe alkyle en C₁ à C₄,
R² représente l'hydrogène ou un groupe alkyle en C₁ à C₄,
comprenant la réaction de cyanate de potassium ou de cyanate de sodium avec une amine de formule (III) 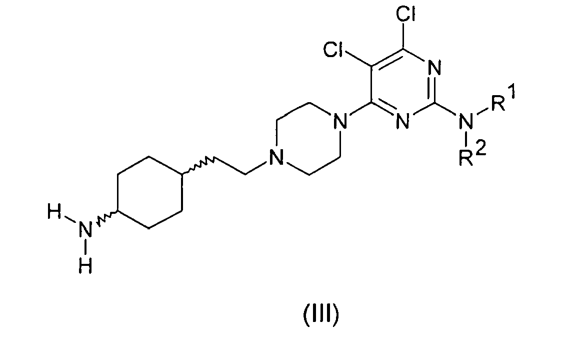 dans laquelle
R¹ représente l'hydrogène ou un groupe alkyle en C₁ à C₄ ;
R² représente un atome d'hydrogène ou un groupe alkyle en C₁ à C₄ ;
et, lorsque c'est approprié, la séparation des énantiomères et/ou diastéréomères, et/ou des isomères cis et/ou trans de composés de formule (I), ou d'intermédiaires pour ceux-ci, par des procédés conventionnels,
et éventuellement ensuite la formation de sels et/ou hydrates et/ou solvates du composé de formule (I).

8. Composition pharmaceutique comprenant un composé de formule (I) selon la revendication 1 et un ou plusieurs véhicules physiologiquement acceptables.

9. Utilisation d'un composé de formule (I) selon la revendication 1 dans la fabrication d'un médicament pour le traitement et/ou la prévention des psychoses, de la pharmacodépendance, des altérations cognitives accompagnant la schizophrénie, des déficits cognitifs légers à modérés, de la démence, des états psychotiques associés à la démence, des troubles de l'alimentation, des troubles déficitaires de l'attention, des troubles d'hyperactivité, de la dépression psychotique, des manies, du trouble bipolaire, des troubles paranoïdes et délirants, des troubles dyskinétiques, de la dépression et des états dépressifs, des troubles de l'anxiété, des dysfonctionnements sexuels, des troubles du sommeil, des vomissements, de l'agressivité, de l'autisme ou de la douleur.

10. Utilisation selon la revendication 9, dans laquelle la psychose est la schizophrénie ou un trouble schizo-affectif ; la pharmacodépendance est une dépendance à l'alcool, à la cocaïne, à la nicotine ou aux opioïdes ; le trouble de l'alimentation est la boulimie nerveuse ; le trouble dyskinétique est la maladie de Parkinson, le parkinsonisme induit par un neuroleptique ou la dyskinésie tardive ; le dysfonctionnement sexuel est un dysfonctionnement érectile.

11. Composé de formule (I) selon la revendication 1, pour utilisation dans le traitement et/ou la prévention de la psychose, de la pharmacodépendance, d'une altération cognitive accompagnant la schizophrénie, des déficits cognitifs légers à modérés, de la démence, des états psychotiques associés à la démence, des troubles de l'alimentation, des troubles déficitaires de l'attention, des troubles d'hyperactivité, de la dépression psychotique, des manies, du trouble bipolaire, des troubles paranoïdes et délirants, des troubles dyskinétiques, de la dépression et des états dépressifs, des troubles de l'anxiété, des dysfonctionnements sexuels, des troubles du sommeil, des vomissements, de l'agressivité, de l'autisme ou de la douleur.

12. Composé selon la revendication 11, dans lequel la psychose est la schizophrénie ou un trouble schizo-affectif ; la pharmacodépendance est une dépendance à l'alcool, à la cocaïne, à la nicotine ou aux opioïdes ; le trouble de l'alimentation est la boulimie nerveuse ; le trouble dyskinétique est la maladie de Parkinson, le parkinsonisme induit par un neuroleptique ou la dyskinésie tardive ; le dysfonctionnement sexuel est un dysfonctionnement érectile.
